(19) Europäisches Patentamt / European Patent Office / Office européen des brevets

(11) **EP 3 890 640 B1**

(12) **EUROPEAN PATENT SPECIFICATION**

(45) Date of publication and mention
of the grant of the patent:
**30.07.2025  Bulletin 2025/31**

(21) Application number: **19893032.3**

(22) Date of filing: **06.12.2019**

(51) International Patent Classification (IPC):
*A61B 34/00* (2016.01)      *A61B 34/30* (2016.01)
*A61B 17/00* (2006.01)      *A61B 17/29* (2006.01)
*A61B 90/00* (2016.01)      *A61B 34/37* (2016.01)

(52) Cooperative Patent Classification (CPC):
**A61B 34/37; A61B 34/00; A61B 34/30;
A61B 34/71;** A61B 2017/00477; A61B 2034/305;
A61B 2090/066

(86) International application number:
**PCT/US2019/064867**

(87) International publication number:
**WO 2020/118149 (11.06.2020 Gazette 2020/24)**

(54) **CONTROLLER FOR CABLE DRIVEN END EFFECTORS**

STEUERUNG FÜR KABELGETRIEBENE ENDEFFEKTOREN

CONTRÔLEUR POUR EFFECTEURS TERMINAUX ENTRAÎNÉS PAR CÂBLE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **06.12.2018  US 201862776285 P**

(43) Date of publication of application:
**13.10.2021  Bulletin 2021/41**

(73) Proprietor: **Covidien LP
Mansfield, MA 02048 (US)**

(72) Inventors:
• **ROCKROHR, Brian**
  **Guilford, Connecticut 06437 (US)**
• **CHENG, Jiqi**
  **Cheshire, Connecticut 06410 (US)**
• **RAHMAN, Farrukh**
  **Middlebury, Connecticut  06762 (US)**
• **MEGLAN, Dwight**
  **Westwood, Massachusetts 02090 (US)**
• **WELLS, Brian**
  **Killingworth, Connecticut 06419 (US)**
• **AGRAWAL, Alok**
  **New Haven, Connecticut 06511 (US)**

(74) Representative: **Maschio & Soames IP Ltd
30 Carlton Crescent
Southampton SO15 2EW (GB)**

(56) References cited:
WO-A1-2018/053305      WO-A1-2019/221754
US-A- 3 952 880          US-A1- 2015 014 114
US-A1- 2015 150 635      US-A1- 2017 143 437
US-A1- 2018 200 894

• I. RIVAS-BLANCO ET AL: "Force-position control
for a miniature camera robotic system for single-
site surgery", 2013 IEEE/RSJ INTERNATIONAL
CONFERENCE ON INTELLIGENT ROBOTS AND
SYSTEMS(ROS), 7 November 2013 (2013-11-07),
pages 3065 - 3070, XP032537356, ISSN:
2153-0858, DOI: 10.1109/IROS.2013.6696790
• BAOLIANG ZHAO ET AL: "Estimating Tool-
Tissue Forces Using a 3-Degree-of-Freedom
Robotic Surgical Tool", JOURNAL OF
MECHANISMS AND ROBOTICS, vol. 8, no. 5,
pages 1 - 10, XP055551827, ISSN: 1942-4302,
DOI: 10.1115/1.4032591

**Description**

CROSS REFERENCE TO RELATED APPLICATIONS

The present application claims the benefit of and priority to U.S. Provisional Patent Application Serial No. 62/776,285, filed on December 6, 2018.

BACKGROUND

Robotic surgical systems such as teleoperative systems are used to perform minimally invasive surgical procedures that offer many benefits over traditional open surgery techniques, including less pain, shorter hospital stays, quicker return to normal activities, minimal scarring, reduced recovery time, and less injury to tissue.

Robotic surgical systems can have a number of robotic arms that move attached instruments or tools, such as an image capturing device, a stapler, an electrosurgical instrument, etc., in response to movement of input devices by a surgeon viewing images captured by the image capturing device of a surgical site. During a surgical procedure, each of the tools is inserted through an opening, either natural or an incision, into the patient and positioned to manipulate tissue at a surgical site. The openings are placed about the patient's body so that the surgical instruments may be used to cooperatively perform the surgical procedure and the image capturing device may view the surgical site.

During the surgical procedure, the tools can include end effectors that are controlled by one or more open loop cables. The end effector can be manipulated by controlling the tension in the cables.

There is a continuing need for improved methods for controlling the tension in the cables to manipulate the end effector.

SUMMARY

The invention is limited by the scope of independent claim 1. Further embodiments are disclosed in the dependent claims. Disclosed methods of treatment by surgery should only be seen as examples of how to use the device and are not claimed. In an aspect of the present disclosure, a method of controlling an end effector of a surgical robot includes receiving a desired pose, generating a motor torque for each motor, transmitting the motor torques for each motor, generating a null torque for each motor, generating a desired torque for each motor, and transmitting the desired torques to an instrument drive unit (IDU) such that the IDU moves the end effector to the desired pose. A primary controller receives the desired pose of the end effector in three degrees-of-freedom (DOF). The primary controller generates the motor torque for each motor of the IDU in response to receiving the desired pose. The primary controller transmits the motor torques which are received in a secondary controller. The secondary controller generates a null torque for each motor of the IDU to maintain tension in cables of a differential drive mechanism of the IDU. The desired torques are generated for each motor of the IDU which torques include a sum of the motor torques and the null torques for each motor.

In aspects, generating the null torque for each motor of the IDU also includes generating a clamping force between jaws of the end effector. Generating the clamping force may include modifying the desired pose such that a jaw angle between the jaws of the end effector is negative. The method may include verifying a position of the jaws of the end effector is less than a clamping threshold before generating the clamping force. The method may include releasing the clamp force when the jaws have a position greater than a releasing threshold. The releasing threshold may be greater than the clamping threshold.

In some aspects, generating the null torque for each motor includes the secondary controller receiving a sensed torque from the IDU. The sensed torque from the IDU may affect the null torque for each motor of the IDU. Generating the null torque for each motor may include adjusting the null torque for each motor in response to a sensed torque of the respective motor. Adjusting the null torque for each motor may include applying a gain to the motor torque of each motor. Generating the null torque for each motor may include adjusting the null torque for a puller motor for each pair of motors of the IDU in response to the sensed torques.

In certain aspects, generating a desired torque for each motor includes a tertiary controller receiving the motor torques and the null torques and combining the motor torques and the null torques into desired torques including the sum of the motor and null torques. The tertiary controller may transmit the desired torques to the IDU. Combining the motor torques and the null torques may include receiving sensed torques from the IDU and applying a gain to the sum of the motor and null torques to determine the desired torques such that the sensed torques approach the sum of the motor and null torques.

In particular aspects, the secondary controller generates the null torque for each motor in response to receiving a motor position for each motor of the IDU. The motor position received by the secondary controller may be in a joint space. A converter positioned between the IDU and the secondary controller converting a motor position from a motor space to the joint space.

In aspects, generating the motor torque for each motor includes calculating the motor torques in a joint space and compensating for friction. The method may include distributing the motor torques in the joint space to each motor before the secondary controller receives the motor torques for each motor.

In another aspect of the present disclosure, a controller for an end effector that is controlled by four cables of an open loop differential drive mechanism includes a primary controller and a secondary controller. The primary controller is configured to receive a desired pose for the end effector in yaw, pitch, and jaw degrees-of-freedom (DOF), to generate a motor torque for each motor of an instrument drive unit (IDU) to position the end effector in the desired pose, and to transmit the motor torques. The secondary controller is configured to receive the motor torques from the primary controller, and to generate null torques for each of the motors of the IDU to maintain tension in the cables of the differential drive mechanism, and an IDU configured to receive desired torques which include a sum of the motor torques and the null torques, and to manipulate the end effector to the desired pose in response to receiving the desired torques.

In aspects, the controller includes a combination controller that is configured to receive the null torques and the motor torques from the secondary controller, to generate the sum of the motor torques and null torques, and to transmit the sum to the IDU.

In another aspect of the present disclosure, an instrument drive unit (IDU) for controlling an end effector controlled by four cables of an open loop differential drive mechanism includes motors, couplers, and torque sensors. The IDU is controlled by a primary controller and a secondary controller. The motors are configured to receive desired torques and to manipulate the end effector to a desired pose in response to receiving the desired torques. The primary controller is configured to receive the desired pose for the end effector on yaw, pitch, and jaw degrees-of-freedom (DOF), to generate a motor torque for the motors to position the end effector in the desired pose, and to transmit the motor torques. The secondary controller is configured to receive the motor torques from the primary controller, to generate null torques for the motors to maintain tension in the cables of the differential drive mechanism, wherein the desired torques include a sum of the motor torques and the null torques.

In another aspect of the present disclosure, an adapter for a surgical tool that defines a longitudinal axis includes, a first drive screw, a first drive nut, a first cable, a first spring, a second drive screw, a second drive nut, a second cable, and a second spring. The first drive screw is longitudinally fixed and configured to rotate about a first screw axis that is parallel to the longitudinal axis and has a threaded portion. The first drive nut is disposed about the threaded portion of the first drive screw and is threadably coupled to the first drive screw such that the first drive nut longitudinally translates in response to rotation of the first drive screw and the first drive screw rotates in response to longitudinal translation of the first drive nut. The first cable has a proximal portion fixed to the first drive nut and a distal portion. The first spring is disposed about the first drive screw and configured to urge the first drive nut in a first longitudinal direction and has a first spring constant.

The second drive screw is longitudinally fixed and configured to rotate about a second screw axis that is parallel to the first screw axis and has a threaded portion. The second drive nut is disposed about the threaded portion of the second drive screw and is threadably coupled to the second drive screw such that the second drive nut longitudinally translates in response to rotation of the second drive screw and the second drive screw rotates in response to longitudinal translation of the second drive nut. The second cable has a proximal portion fixed to the second drive nut and a distal portion.

The distal portions of the first and second cables are operatively coupled to one another such that translations of the distal portions oppose one another. The second spring is disposed about the second drive screw and configured to urge the second drive nut in the first direction and has a second spring constant. The second spring biasedsuch that the second spring translates the second drive nut and the second cable in the first direction such that the second cable translates the first cable and the first drive nut in a second direction opposite the first direction and against the bias of the first spring such that the tool is biased towards a predetermined pose.

In aspects, the first screw includes a first proximal head that is configured to interface with a first motor and the second screw includes a second proximal head that is configured to interface with a second motor. The first direction may be proximal and the second direction may be distal. The first drive nut may define a first slot with the proximal portion of the first cable fixed in the first slot. The second spring constant may be larger than the first spring constant.

In another aspect of the present disclosure a surgical tool includes an elongate shaft, an end effector, and an adapter. The elongated shaft defines a longitudinal axis and has a proximal end and a distal end. The end effector is supported adjacent the distal end of the elongate shaft and includes a first jaw and a second jaw movable in pitch, yaw, and jaw DOFs. The adapter supports the proximal end of the elongate shaft and includes a first drive screw, a first drive nut, a first cable, a first spring, a second drive screw, a second drive nut, a second cable, and a second spring. The first drive screw is longitudinally fixed and configured to rotate about a first screw axis that is parallel to the longitudinal axis and has a threaded portion. The first drive nut is disposed about the threaded portion of the first drive screw and is threadably coupled to the first drive screw such that the first drive nut longitudinally translates in response to rotation of the first drive screw and the first drive screw rotates in response to longitudinal translation of the first drive nut. The first cable extends through the elongate shaft and has a proximal portion fixed to the first drive nut and a distal portion secured to the end effector. The first spring is disposed about the first drive screw and configured to urge the first drive nut in a first longitudinal direction and has a first spring constant.

The second drive screw is longitudinally fixed and configured to rotate about a second screw axis that is parallel to the first screw axis and has a threaded portion. The second drive nut is disposed about the threaded portion of the second drive screw and is threadably coupled to the second drive screw such that the second drive nut longitudinally translates in response to rotation of the second drive screw and the second drive screw rotates in response to longitudinal translation of the second drive nut. The second cable extends through the elongate shaft and has a proximal portion fixed to the second drive nut and a distal portion secured to the end effector.

The distal portions of the first and second cables are operatively coupled to one another such that translations of the distal portions oppose one another. The second spring is disposed about the second drive screw and configured to urge the second drive nut in the first direction and has a second spring biased such that the second spring translates the second drive nut and the second cable in the first direction such that the second cable translates the first cable and the first drive nut in a second direction opposite the first direction and against the bias of the first spring such that the tool is biased towards a predetermined pose.

In aspects, the distal portions of the first and second cables are each coupled to the first jaw.

In some aspects, the adapter includes a third drive screw, a third drive nut, a third cable, a third spring, a fourth drive screw, a fourth drive nut, a fourth cable, and a fourth spring. The third drive screw is longitudinally fixed within the adapter and configured to rotate about a third screw axis parallel to the longitudinal axis and has a threaded portion. The third drive nut is disposed about the threaded portion of the third drive screw and is threadably coupled to the third drive screw such that the third drive nut longitudinal translates in response to rotation of the third drive screw and the third drive screw rotates in response to longitudinal translation of the third drive nut. The third cable extends through the elongate shaft and has a proximal portion that is fixed to the third drive nut and a distal portion secured to the end effector. The third spring is disposed about the third drive screw and is configured to urge the third drive nut in a third longitudinal direction and has a third spring constant.

The fourth drive screw is longitudinally fixed within the adapter and is configured to rotate about a fourth screw axis that is parallel to the third screw axis and has a threaded portion. The fourth drive nut is disposed about the threaded portion of the fourth drive screw and is threadably coupled to the fourth drive screw such that the fourth drive nut longitudinally translates in response to rotation of the fourth drive screw and the fourth drive screw rotates in response to longitudinal translation of the fourth drive nut. The fourth cable extends through the elongate shaft and has a proximal portion fixed to the fourth drive nut and a distal portion secured to the end effector. The distal portions of the third and fourth cables are operatively coupled to one another such that translations of the distal portions oppose one another. The fourth spring is disposed about the fourth drive screw, is configured to urge the fourth drive nut in the first direction, and has a fourth spring biased such that the fourth spring translates the fourth drive nut and the fourth cable in the first direction. The fourth cable translating the third cable and the third drive nut in the second direction and against the bias of the third spring such that the end effector is biased towards the predetermined pose.

In certain aspects, the distal portion of the first cable is secured to a first side of the first jaw, the distal portion of the second cable is secured to a second side of the first jaw, the distal portion of the third cable is secured to the second side of the second jaw, and the distal portion of the fourth cable is secured to the first side of the second jaw such that the first and fourth cables are disposed on the same side of the first and second jaws, respectively, and the second and third cables are disposed on the same side of the first and second jaws, respectively. The end effector may include a yoke and a clevis. The clevis may be fixed to the distal end of the elongate shaft and the yoke may be pivotally coupled to the clevis about a first axis perpendicular to and intersected by the longitudinal axis. The jaw may be pivotally coupled to the yoke about a second axis perpendicular to the first axis. The first jaw may have a first spindle pivotal about the second axis and the second jaw may have a second spindle pivotal about the second axis. The distal portions of the first and second cables may be secured to opposite sides of the first spindle and the distal portions of the third and fourth cables may be secured to opposite sides of the second spindle.

In particular aspects, the second and fourth springs are configured to maintain the tool in a pose with the first and second jaws in a closed position, the first and second jaws longitudinally aligned with the longitudinal axis, and the yoke aligned with the longitudinal axis. The first, second, third, and fourth cables may be configured to manipulate the pose of the end effector in pitch, yaw, and jaw DOFs.

In another aspect of the present disclosure, a surgical tool configured to selectively connect to a drive unit includes an elongate shaft, an end effector, a first cable, a second cable, a third cable, a fourth cable, and an adapter. The elongate shaft defines a longitudinal axis and has a proximal end and a distal end. The end effector is supported adjacent the distal end of the elongate shaft and includes a first jaw and a second jaw. The first cable extends through the elongate shaft and has a distal portion secured to a first side of the first jaw. The second cable extends through the elongate shaft and has a distal portion secured to a second, opposite side of the first jaw. The third cable extends through the elongate shaft and has a distal portion secured to the second side of the second jaw.

The fourth cable extends through the elongate shaft and has a distal portion secured to the first side of the second jaw. The adapter supports the proximal end of the elongate shaft and is configured to selectively connect to a drive unit. The adapter includes a differential drive mechanism configured to manipulate proximal portions of each of the first, second, third, and fourth cables to manipulate the end effector in pitch, yaw, and jaw DOFs. Each of the first, second, third, and fourth cables are biased proximally and configured to maintain the end effector in a desired pose with the tool is disconnected from a drive unit.

In aspects, the adapter urges each of the first and third cables proximally with a first force and urges each of the second and fourth cable proximally with a second force greater than the first force. The desired pose may be straight in pitch and yaw with the first and second jaws in a closed position such that the first and second jaws are closed and aligned with the longitudinal axis. Alternatively, the desired pose may be straight in pitch and yaw with the first and second jaws in an open position such that the first and second jaws are spaced apart from one another and aligned with the longitudinal axis.

In some aspects, the end effector includes a yoke and a clevis. The clevis may be fixed to the distal end of the elongate shaft. The yoke is pivotally coupled to the clevis about a first axis that is perpendicular to and intersected by the longitudinal axis. The jaws may be pivotally coupled to the yoke about a second axis perpendicular to the first axis. The first jaw has a first spindle pivotal about the second axis and the second jaw has a second spindle pivotal about the second axis.

In certain aspects, the differential drive mechanism includes a drive screw, a nut threadably coupled to the drive screw, and a spring biasing the nut proximally for each of the first, second, third, and fourth cables with the proximal portion of each of the first, second, third, and fourth cables fixed to a respective nut.

Further, to the extent consistent, any of the aspects described herein may be used in conjunction with any or all of the other aspects described herein.

## Brief Description of the Drawings

Various aspects of the present disclosure are described hereinbelow with reference to the drawings, which are incorporated in and constitute a part of this specification, wherein:

FIG. 1 is a schematic illustration of a user interface and a surgical robot of a robotic surgical system in accordance with the present disclosure;

FIG. 2 is a side, perspective view of an arm of the robotic system of FIG. 1 including an IDU, an adapter assembly, and a tool having an end effector;

FIG. 3 is a rear, perspective view of the tool of FIG. 2;

FIG. 4 is a cross-sectional view taken along the section line 3-3 of FIG. 3;

FIG. 5 is a perspective view showing internals of an adapter of the tool of FIG. 3;

FIG. 6 is an enlarged, perspective view of the end effector of the tool of FIG. 2 with jaws in a straight, closed configuration;

FIG. 7 is a perspective view of the end effector of FIG. 6 with the end effector rotated in a positive yaw DOF;

FIG. 8 is a perspective view of the end effector of FIG. 6 with the end effector rotated in a positive pitch DOF;

FIG. 9 is a perspective view of the end effector of FIG. 6 with the end effector rotated in a positive jaw DOF;

FIG. 10 is a perspective view of the end effector of FIG. 6 with the end effector rotated in the positive pitch DOF, the positive yaw DOF, and the positive jaw DOF;

FIG. 11 is a schematic of a positional controller in accordance with an embodiment of the present disclosure;

FIG. 12 is a schematic of an overall controller in accordance with an embodiment of the present disclosure;

FIG. 13 is a schematic of another overall controller in accordance with an embodiment of the present disclosure; and

FIG. 14 is a schematic of another overall controller in accordance with an embodiment of the present disclosure.

## DETAILED DESCRIPTION

Embodiments of the present disclosure are now described in detail with reference to the drawings in which like reference numerals designate identical or corresponding elements in each of the several views. As used herein, the term "clinician" refers to a doctor, a nurse, or any other care provider and may include support personnel. Throughout this description, the term "proximal" refers to the portion of the device or component thereof that is closer to the clinician or surgical robot manipulating the device or component and the term "distal" refers to the portion of the device or component thereof that is farther from the clinician or surgical robot manipulating the device.

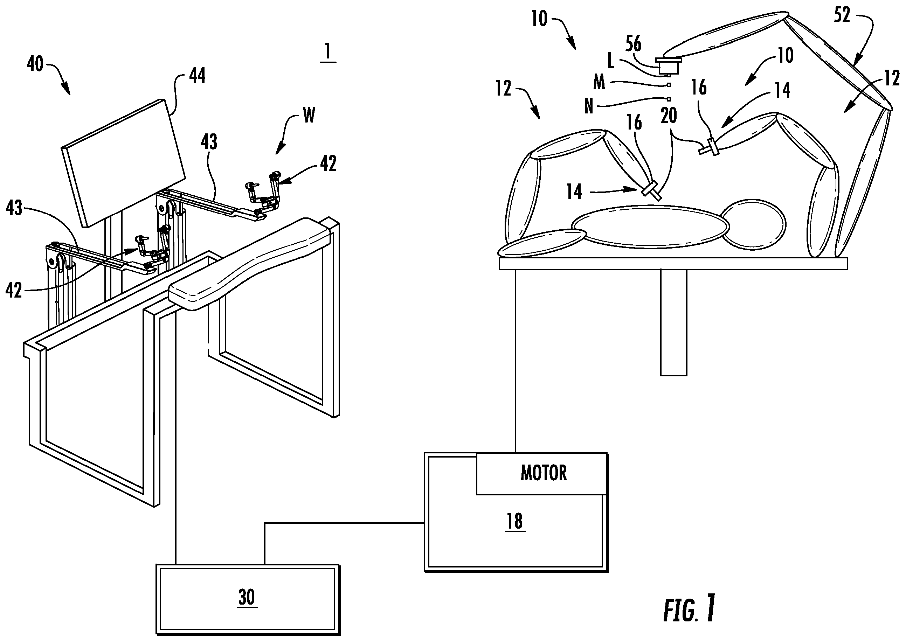

Referring to FIG. 1, a robotic surgical system 1 in accordance with the present disclosure is shown generally as a robotic system 10, a processing unit 30, and a user interface 40. The robotic system 10 generally includes linkages or arms 12 and a robot base 18. The arms 12 moveably support a tool 20 which is configured to act on tissue. The arms 12 each have an end 14 that supports a tool 20 which is configured to act on tissue. In addition, the ends 14 of the arms 12 may include an imaging device 16 for imaging a surgical site. The user interface 40 is in communication with robot base 18 through the processing unit 30.

The user interface 40 includes a display device 44 which is configured to display three-dimensional images. The display device 44 displays three-dimensional images of the surgical site which may include data captured by imaging devices 16 positioned on the ends 14 of the arms 12 and/or include data captured by imaging devices that are positioned about the surgical theater (e.g., an imaging device positioned within the surgical site, an imaging device positioned adjacent the patient, imaging device 56 positioned at a distal end of an imaging linkage or arm 52). The imaging devices (e.g., imaging devices 16, 56) may capture visual images, infra-red images, ultrasound images, X-ray images, thermal images, and/or any other known real-time images of the surgical site. The imaging devices transmit captured imaging data to the processing unit 30 which creates three-dimensional images of the surgical site in real-time from the imaging data and transmits the three-dimensional images to the display device 44 for display.

The user interface 40 also includes input handles 42 which are supported on control arms 43 which allow a clinician to manipulate the robotic system 10 (e.g., move the arms 12, the ends 14 of the arms 12, and/or the tools 20). Each of the input handles 42 is in communication with the processing unit 30 to transmit control signals thereto and to receive feedback signals therefrom. Additionally or alternatively, each of the input handles 42 may include input devices (not shown) which allow the surgeon to manipulate (e.g., clamp, grasp, fire, open, close, rotate, thrust, slice, etc.) the tools 20 supported at the ends 14 of the arms 12.

Each of the input handles 42 is moveable through a predefined workspace to move the ends 14 of the arms 12 within a surgical site. The three-dimensional images on the display device 44 are orientated such that movement of the input handle 42 moves the ends 14 of the arms 12 as viewed on the display device 44. It will be appreciated that the orientation of the three-dimensional images on the display device may be mirrored or rotated relative to a view from above the patient. In addition, it will be appreciated that the size of the three-dimensional images on the display device 44 may be scaled to be larger or smaller than the actual structures of the surgical site permitting a clinician to have a better view of structures within the surgical site. As the input handles 42 are moved, the tools 20 are moved within the surgical site as detailed below. As detailed herein, movement of the tools 20 may also include movement of the ends 14 of the arms 12 which support the tools 20.

For a detailed discussion of the construction and operation of a robotic surgical system 1, reference may be made to U.S. Patent No. 8,828,023.

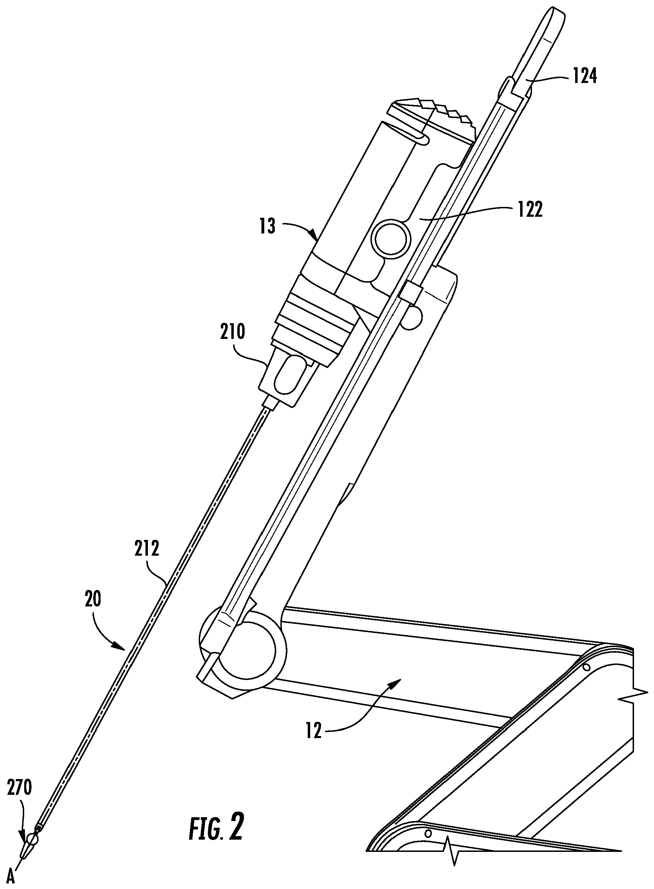

With reference to FIG. 2, a portion of an exemplary arm 12 of the surgical robot 10 of FIG. 1. The arm 12 includes a carriage 122 that is translatable along a rail 124. An instrument drive unit (IDU) 13 is secured to the carriage 122. The IDU 13 has one or more motors (not shown) that are configured to control a tool 20 as detailed below. For a detailed discussion of an exemplary IDU including one or more motors, reference may be made to U.S. Patent Publication No. 2018/0153634.

The tool 20 includes an adapter 210, an elongate shaft 212 that extends distally from the adapter 210, and an end effector 270 supported by a distal portion of the elongate shaft 212. The adapter 210 is releasably coupled to the IDU 13 such that the tool 20 receives input from the IDU 13.

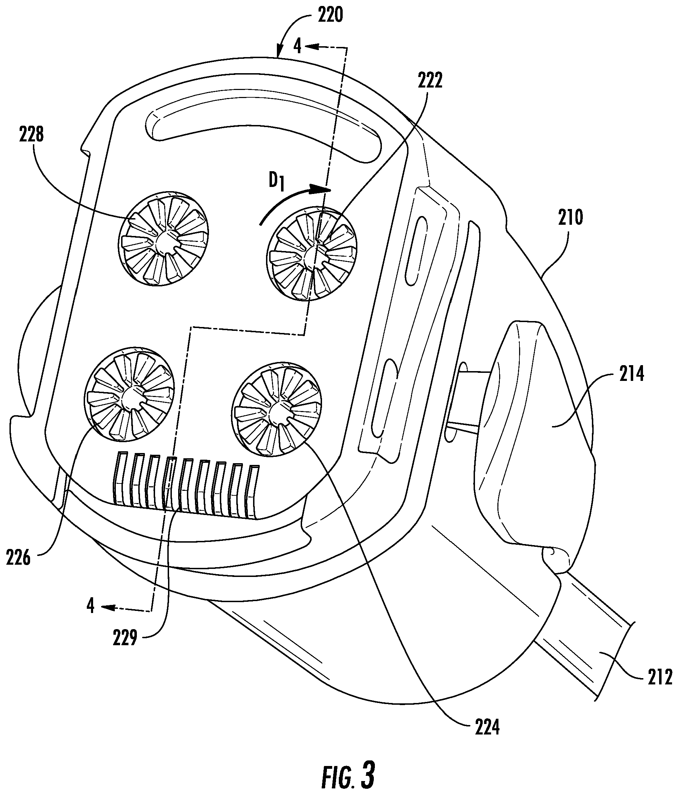

With additional reference to FIG. 3, the adapter 210 includes an IDU interface 220 including a first motor interface 222, a second motor interface 224, a third motor interface 226, a fourth motor interface 228, and a control interface 229. Each of the motor interfaces 222-228 is configured to mechanically couple to a respective motor of the IDU 13. The motor interfaces 222, 224, 226, 228 are arranged about the longitudinal axis A-A of the shaft 212. The motor interfaces 222, 224, 226, 228 may from a rectangle or square in a plane orthogonal to the longitudinal axis A-A of the shaft 212. The control interface 229 is configured to couple to a control interface of the IDU 13 or the carriage 122 to receive instructions from the surgical robot 10 and/or the processing unit 30 and/or to transmit data to the surgical robot 10 and/or the processing unit 30.

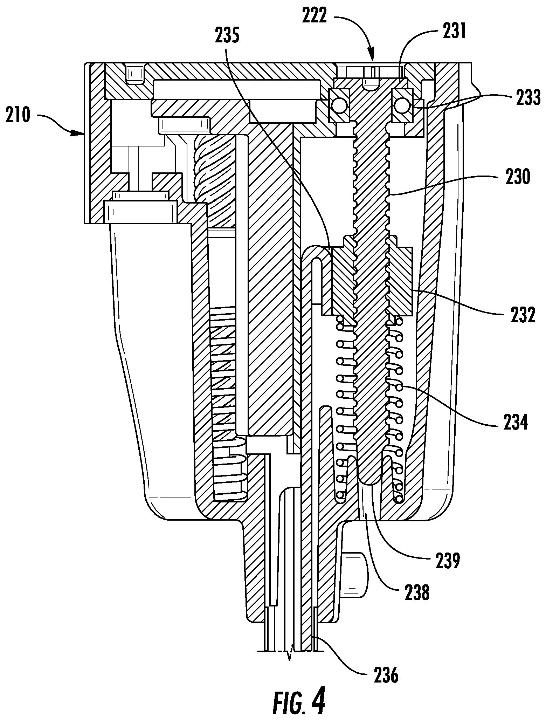

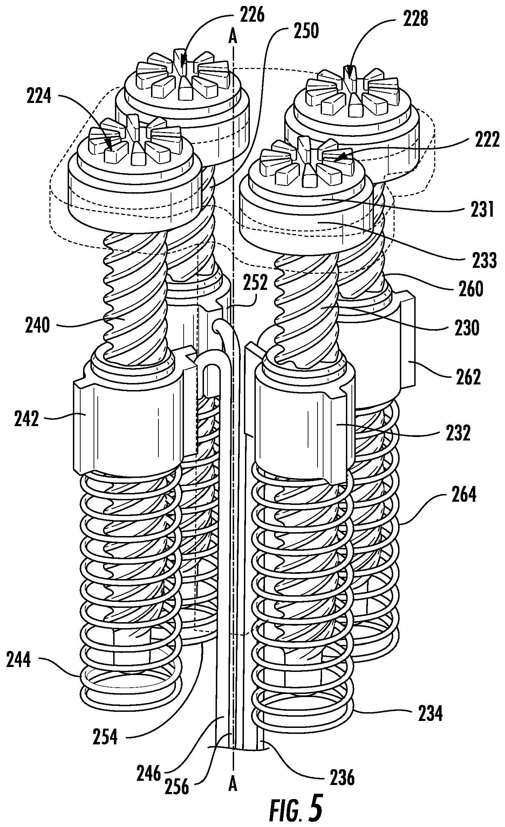

Referring to FIGS. 4 and 5, the first motor interface 222 includes a first drive screw 230, a first drive nut 232, a first spring 234, and a first cable 236. The first drive screw 230 includes a first head 231 and a distal nub 239. The first head 231 may have radial teeth, a slot, a female connector, a male connector, or any suitable interface for coupling coaxial rotating shafts such that the first head 231 is configured to mechanically couple the first drive screw 230 to a motor of the IDU 13.

The first drive screw 230 is supported within the adapter 210 by a first bearing 233 positioned adjacent the first head 231. The distal nub 239 is received within a first opening 238 defined by the adapter 210 such that the first bearing 233 and the distal nub 239 support the first drive screw 230 within the adapter 210 and enable the first drive screw 230 to rotate about its longitudinal axis, maintain the longitudinal axis of the first drive screw 230 parallel to a longitudinal axis of the elongate shaft 212, and prevent the first drive screw 230 from translating along its longitudinal axis.

The first drive nut 232 is disposed over a threaded portion of the first drive screw 230 such that the first drive nut 232 and the first drive screw 230 are threadably coupled with one another. Specifically, as the first drive screw 230 is rotated in a first direction, e.g., clockwise rotation about the longitudinal axis of the drive screw as shown with arrow $D_1$ in FIG. 3, the first drive nut 232 translates proximally along the first drive screw 230 towards the first head 231 and when the first drive screw 230 is rotated in a second direction opposite the first direction, e.g., counter-clockwise, the first drive nut 232 is translated distally along the first drive screw 230 away from the first head 231. The first drive nut 232 defines a first slot 235 that receives a portion of the first cable 236 such that as the first drive nut 232 translates along the first drive screw 230, the first cable 236 cooperates with translation of the first drive nut 232. Specifically, the first cable 236 is retracted as the first drive nut 232 translates proximally and the first cable 236 is relaxed as the first drive nut 232 translates distally.

The first spring 234 is disposed about the first drive screw 230 distal of the first drive nut 232 and engages a distal surface of the first drive nut 232. The first spring 234 is supported within the adapter 210 such that the first spring 234 urges the first drive nut 232 proximally. The first spring 234 may have a spring constant large enough to urge the first drive nut 232 proximally such that the first drive screw 230 is rotated in the first direction absent a force applied to the first head 231. The first spring 234 may have a constant or progressive spring constant.

The second motor interface 224 includes a second drive screw 240, a second spring 244, and a second cable 246; the third motor interface 226 includes a third drive screw 250, a third spring 254, and a third cable 256; and the fourth motor interface 228 includes a fourth drive screw 260, a fourth spring 264, and a fourth cable 266. The drive screws 240, 250, 260, the springs 244, 254, 264, and the cables 246, 256, 266 are similar to the first drive screw 230, the first spring 234, and the first cable 236, respectively, detailed above and will not be detailed herein for brevity except where the differences are relevant to the function of the tool 20.

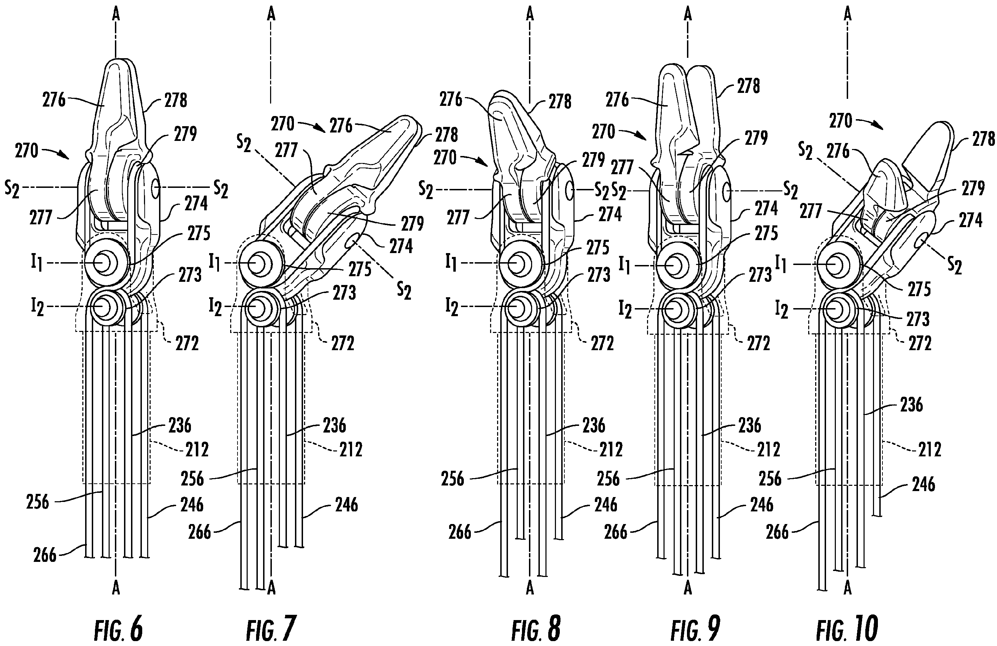

With reference to FIGS. 6-10, the cables 236, 246, 256, 266 extend through the shaft 212 and are connected to the end effector 270 to control movement of the end effector 270 in three degrees-of-freedom (DOF), e.g., yaw, pitch, and jaw. The end effector 270 includes a clevis 272, a yoke 274, a first jaw 276, and a second jaw 278. The clevis 272 includes a first idler 273 and the yoke 274 includes a second idler 275 distal of the first idler 273. The first and second idlers 273, 275 each define an idler axis $I_1$, $I_2$ that is perpendicular to the longitudinal axis A-A of the shaft 212 and parallel to one another.

The first jaw 276 includes a first spindle 277 and the second jaw 278 includes a second spindle 279. The first spindle 277 and the second spindle 279 each define a spindle axis $S_1$, $S_2$ that is perpendicular to the longitudinal axis A-A of the shaft 212, when the shaft 212 is in a straight configuration as shown in FIG. 5, and perpendicular to the second idler axis $I_2$. The first and second spindle axes $S_1$, $S_2$ may be coaxial with one another.

The clevis 272 pivotally supports the yoke 274 about the second idler axis $I_2$ in a yaw DOF. The yoke 274 pivotally supports the first and second jaws 276, 278 about the first and second spindle axes $S_1$, $S_2$ in pitch and jaw. Specifically, when the first and second jaws 276, 278 pivot about the first and second spindle axes $S_1$, $S_2$ in the same direction in concert with one another, the first and second jaws 276, 278 move in a pitch DOF. Alternatively, when the first and second jaws 276, 278 pivot about the first and second spindle axes $S_1$, $S_2$ in opposite directions or independent of one another, the first and second jaws 276, 278 move in a jaw DOF. The first and second jaws 276, 278 can move in the same direction but at different speeds, e.g., not in concert, such that the first and second jaws 276, 278 move in both the pitch DOF and the jaw DOF.

Continuing to refer to FIG. 6, the cables 236, 246, 256, 266 wrap around the first and second idlers 273, 275 and are secured to a respective one of the first and second spindles 277, 279. The first and second cables 236, 246 are secured to opposite sides of the first spindle 277 of the first jaw 276. Specifically, the first cable 236 may be secured to a top side of the first spindle 277 and the second cable 246 may be secured to a bottom side of the first spindle 277. The first and second cables 236, 246 may form a continuous monolithic cable with one another that wraps about the first spindle 277. The third and fourth cables 256, 266 are secured to opposite sides of the second spindle 279 of the second jaw 278. Specifically, the third cable 256 may be secured to a bottom side of the second spindle 279 and the fourth cable 266 may be secured to a bottom side of the second spindle 279. The third and fourth cables 256, 266 may form a continuous monolithic cable with one another that wraps about the second spindle 279.

The first and second idlers 273, 275 may define a separate groove to guide each of the cables 236, 246, 256, 266 around the respective idler 273, 275 such that the cables 236, 246, 256, 266 are secured within the respective groove without interfering with the other cables 236, 246, 256, 266.

Continuing to refer to FIGS. 6-10, displacement of the cables 236, 246, 256, 266 are used as a differential drive to control the end effector 270 in the yaw DOF, the pitch DOF, and the jaw DOF. Initially referring to FIG. 6, the end effector 270 is in a straight configuration in which the cables 236, 246, 256, 266 are in a neutral position relative to one another. In the neutral position, the tension in each of the cables 236, 246, 256, 266 may be substantially equal to one another. To move the end effector 270 in a positive yaw direction, the third and fourth cables 256, 266, which are each secured to the second spindle 278, are each retracted a distance and the first and second cables 236, 246, which are each secured to the first spindle 276, are each extended or relaxed the same distance as shown in FIG. 7. As shown, the positive yaw direction is pivoting the yoke 274 to the right and the negative yaw direction is pivoting the yoke 274 to the left. To move the end effector 270 in the negative yaw direction, the first and second cables 236, 246 are retracted and the third and fourth cables 256, 266 are extended or relaxed the same distance that the first and second cables 236, 246 are retracted.

Referring now to FIG. 8, to move the end effector 270 in a positive pitch direction, upwardly as shown, the first and fourth cables 236, 266, which are each secured to the top side of different spindles 276, 278, are retracted a distance and the second and third cables 246, 256, which are each secured to the bottom side of different spindles 276, 278, are relaxed the same distance. To move the end effector 270 in the negative pitch direction, downwardly as shown, the second and third cables 246, 256 are retracted a distance and the first and fourth cables 236, 266 are relaxed the same distance.

With reference to FIG. 9, to move the end effector 270 in a positive jaw direction, to pivot the jaws 276, 278 apart from one another, the first and third cables 236, 256, which are each secured to different sides of different spindles 276, 278, are retracted and the second and fourth cables 246, 266, which are secured to different sides of different spindles 276, 278, are relaxed. The first and third cables 236, 256 may be retracted the same distances or different distances. However, the second cable 246 is relaxed the same distance that the first cable 236 is retracted and the fourth cable 266 is relaxed the same distance that the third cable 256 is retracted. To move the end effector 270 in the negative jaw direction, e.g., to move the jaws 276, 278 towards one another, the second and fourth cables 246, 266 are retracted and the first and third cables 236, 256 are relaxed.

It is contemplated that one jaw, e.g., second jaw 278, may be stationary as the other jaw, e.g., first jaw 276, is pivoted such that the end effector 270 moves in the jaw direction. To move the end effector 270 in the positive jaw direction with second jaw 278 stationary, the first cable 236 can be retracted and the second cable 246 is relaxed an equal amount while the third and fourth cables 256, 266 remain stationary. Similarly to move the end effector 270 in the positive jaw direction with the first jaw 276 stationary, the third cable 236 is retracted and the fourth cable 266 is relaxed and equal amount while the first and second cables 236, 246 remain stationary. Each of these movements can be reversed to move the end effector 270 in the negative jaw direction with one of the jaws 276, 278 stationary.

Referring now to FIG. 10, the end effector 270 may be moved in more than one DOF sequentially or simultaneously. For example, the end effector 270 may be moved from the straight configuration (FIG. 6) to the position in FIG. 10 by retracting the fourth cable 266 and relaxing the second cable 246 while substantially maintaining the position of the first and third cables 236, 256 to move the end effector 270 in the positive yaw, pitch, and jaw directions simultaneously.

While several movements of the end effector 270 in the yaw DOF, the pitch DOF, and the jaw DOF are described above, these are meant to be exemplary movements and not an exhaustive list of all possible movements or combination of movements of the end effector 270 in the yaw, pitch, and jaw DOFs.

Referring to FIGS. 2, 5, and 6, it may be desirable to maintain the end effector 270 in a known or neutral position when the tool 20 is disconnected from the IDU 13. By maintaining the tool 20 in a known position, the robotic system 1 can know the position or pose of the end effector 270 when the tool 20 is connected to the IDU 13 without the need for running a calibration sequence. This may reduce the time required to calibrate a surgical robot 10 each time a new tool 20 is attached. In addition, the robotic system 10 can know the position of the end effector 270 without requiring absolute encoders which may reduce the cost of each tool 20. This known pose may be stored in memory (not shown) of the tool 20 and communicated to the robotic system 1 through the control interface 29 when the tool 20 is attached to the surgical robot 10.

To maintain the end effector 270 in a known or neutral pose, the springs 234, 244, 254, 264 can be used as pretension springs. For some tools, e.g., clip appliers or staplers, it may be beneficial to have end effectors maintained in a fully open, or fully positive jaw, configuration when the tool 20 is disconnected from the IDU 13. For example, clip appliers and staplers may need to be in an open position to load clips or staples into a jaw of the end effector. For such instruments, the first and third springs 234, 254 each have a large, first spring constant and the second and fourth springs 244, 264 each have a smaller, second spring constant such that the first and third springs 234, 254 overpower the second and fourth springs 244, 264 to move the jaws 276, 278 towards the fully open configuration. In addition, as the second and fourth springs 244, 264 maintain tension in the second and third cables 246, 266, such that the end effector 270 remains straight with respect to the yaw and pitch directions. Additionally or alternatively, the first and third springs 234, 254 may have the same or substantially the same spring constant as the second and fourth springs 244, 264 and be biased such that the first and third springs 234, 254 overpower the second and fourth springs 244, 264 to move the jaws towards the fully open configuration.

Alternatively, it may be beneficial for some end effectors to be maintained in a fully closed, or fully negative jaw, configuration when the tool 20 is disconnected from the IDU 13. For such instruments, the second and fourth springs 244, 264 each have a large, first spring constant and the first and third springs 234, 254 each have a smaller, second spring constant such that the second and fourth springs 244, 264 overpower the first and third springs 234, 254 to move the jaws 276, 278 towards the full closed configuration. In addition, as the first and third springs 234, 254 maintain tension in the first and third cables 236, 256, the end effector 270 remains straight with respect to the yaw and pitch directions. Additionally or alternatively, the second and fourth springs 244, 264 may have the same or substantially the same spring constant as the first and third springs 234, 254 and be biased such that the second and fourth springs 244, 264 overpower the first and third springs 234, 254 to move the jaws towards the fully closed configuration.

The springs 234, 244, 254, 256 may be configured to maintain the tool 20 in other neutral positions which may be beneficial for a tool 20 with a particular end effector 270. The configuration of the tool 20 may be maintained by varying spring constants of one or more of the springs 234, 244, 254, 256 and/or biasing one or more of the springs 234, 244, 254, 256. The neutral position of the tool 20 may be communicated to the surgical robot 10 and/or the processing unit 30 through the control interface 229, when the tool 20 is connected to the IDU 13.

A forward kinematic model to relate measured motor positions of the motors of the IDU 13 to calculated yaw, pitch, and jaw positions of the end effector 270 is required to determine a pose of the end effector 270. This is different than traditional robots where joint angles are generally directly measured by position sensors, such as potentiometers or encoders. However, as space is extremely limited within the end effector 270, it is difficult to place encoders, potentiometers, or other devices which directly measure the pose of the end effector 270 within the end effector 270. Thus, it is advantageous to calculate the pose of the end effector from the measured position of the motors of the IDU 13. In addition, inaccuracies of the calculated pose can be compensated for by observations of the end effector 270 within the surgical site by a clinician interfacing with the robotic surgical system 1 (FIG. 1).

Kinematic control methods for controlling the end effector 270 in the yaw DOF, the pitch DOF, and the jaw DOF are described below with reference to the tool 20 detailed in FIGS. 2-6. In the model below, the first jaw 276 will be referred to as jaw a and the second jaw 278 will be referred to as jaw b to avoid confusion with integers used in the following equations. It will be appreciated that the arm 12 of the surgical robot 10 is configured to move the end effector 270 in an additional four DOFs such that the end effector 270 is moveable in six DOFs and the jaw DOF.

As detailed above, the jaws "a, b" are moved by displacing one or more of the cables 236, 246, 256, 266 with the motors (not shown) of the IDU 13. A first torque $\tau_a$ is the torque applied to the first jaw "a" and a second torque $\tau_b$ is the torque applied to the second jaw "b". As shown, the first and second jaws "a, b" are typically mirror images of each other with only minor differences and both of the first and second jaws "a, b" pivot about the same pin or axis, e.g., spindle axes $S_1$, $S_2$. As such, the first and second torques $\tau_a$, $\tau_b$ can be represented by the following equations:

$$\begin{cases} \tau_a = m_a\,\ddot{\theta}_a + c_a\,\dot{\theta}_a + \mu_1(f_1 + f_2)sgn(\dot{\theta}_a)r_p \\ \tau_b = m_b\,\ddot{\theta}_b + c_b\,\dot{\theta}_b + \mu_1(f_3 + f_4)sgn(\dot{\theta}_b)r_p \end{cases} \qquad (1)$$

where $\mu_1$ is the coefficient of friction between the respective jaw "a, b" and the pin (not explicitly shown) that the respective jaw "a, b" is pivotal about, $c_a$, $c_b$ are dampening terms, and "m" is an inertial term. The dampening terms $c_a$, $c_b$ and the inertial term "m" are a function of a joint angle $\theta$.

In addition, the first jaw a is directly articulated by the first and second cables 236, 246 and the second jaw b is directly articulated by the third and fourth cables 256, 266 with the first and fourth cables 236, 266 being secured to the top side of the respective jaw "a, b", and the second and third cables 246, 256 being secured to the bottom side of the respective jaw "a, b". As such the torque $\tau_a$, $\tau_b$ can also be represented by the following equations:

$$\begin{cases} \tau_a = (f_1 - f_2)r_p \\ \tau_b = (f_4 - f_3)r_p \end{cases} \qquad (2)$$

If each jaw "a, b" is considered separately, the inertial term "m" and the dampening term "c" are independent of the joint angle $\theta$. Since the jaws "a, b", are substantial mirrors of each other, it can be assumed that the combined term of the inertial term "m" and the dampening term "c" is twice the respective term from a single jaw such that $m_p \equiv 2m_a \approx 2m_b$ and $c_p \equiv 2c_a \approx 2c_b$. Thus, combining Equation (2) into Equation (1), and rearranging the equation, results in the following:

$$\begin{cases} (f_1 - f_2)r_p - \mu_1(f_1 + f_2)sgn(\dot{\theta}_a)r_p + (f_4 - f_3)r_p - \mu_1(f_3 + f_4)sgn(\dot{\theta}_b)r_p = m_p\dfrac{\ddot{\theta}_a + \ddot{\theta}_b}{2} + c_p\dfrac{\dot{\theta}_a + \dot{\theta}_b}{2} \\ 2\left\{(f_1 - f_2)r_p - \mu_1(f_1 + f_2)sgn(\dot{\theta}_a)r_p - (f_4 - f_3)r_p + \mu_1(f_3 + f_4)sgn(\dot{\theta}_b)r_p\right\} = m_p(\ddot{\theta}_a - \ddot{\theta}_b) + c_p(\dot{\theta}_a - \dot{\theta}_b) \end{cases} \qquad (3)$$

Next, a synthetic joint of pitch, for the pitch DOF, is formed by combining jaw "a" and jaw "b". As noted above, the inertial term $"m_p"$ and the dampening term $"c_p"$ of the synthetic pitch joint are twice that of the dampening terms and inertial terms for each of the individual jaws "a, b". The pitch angle $\theta_p$ is defined by a line bisecting an angle between the first and second jaws "a, b". thus, the articulation torque in pitch is defined as:

$$\begin{cases} \tau_p \equiv (f_1 - f_2)r_p - \mu_1(f_1 + f_2)sgn(\dot{\theta}_a)r_p + (f_4 - f_3)r_p - \mu_1(f_3 + f_4)sgn(\dot{\theta}_b)r_p \\ \theta_p \equiv \dfrac{\theta_a + \theta_b}{2} \end{cases} \tag{4}$$

A synthetic joint of jaw, for the jaw DOF, is formed by the combination of the first and second jaws "a, b" with j oint pitch as:

$$\begin{cases} \tau_j \equiv 2\left\{ (f_1 - f_2)r_p - \mu_1(f_1 + f_2)sgn(\dot{\theta}_a)r_p - (f_4 - f_3)r_p + \mu_1(f_3 + f_4)sgn(\dot{\theta}_b)r_p \right\} \\ \theta_j \equiv \theta_a - \theta_b \end{cases} \tag{5}$$

Substituting Equations (3) and (4) into Equation (2) provides basic dynamic equations for the synthetic joint of pitch and the synthetic joint of jaw as:

$$\begin{cases} \tau_p = m_p \ddot{\theta}_p + c_p \dot{\theta}_p \\ \tau_j = m_p \ddot{\theta}_j + c_p \dot{\theta}_j \end{cases} \tag{6}$$

As detailed above, Equations (4) and (5) describe the relationship between forces in the cables 236, 246, 256, 266 and the articulation torque for the synthetic joints of pitch and jaw. The final DOF of the end effector 270 is the yaw DOF which is articulated by all four cables. For the joint of yaw, the dynamic equation depends on the configuration of the end effector 270 in pitch and jaw. Generally, in medical applications the motion in pitch and jaw are quick. As such, for simplicity, this dependency can be ignored to provide:

$$(f_1 + f_2)r_y - (f_4 + f_3)r_y = m_y \ddot{\theta}_y + c_y \dot{\theta}_y + \mu_2(f_1 + f_2 + f_3 + f_4)sgn(\dot{\theta}_y)r_y \tag{7}$$

where inertial term $"m"$ and Coriolis and centrifugal term "c" are lumped parameters that take the pitch and jaw joints into account and $\mu_2$ is a friction coefficient of the yoke 274 about the idler 275. Equation (7) is rearranged to define articulating torque of yaw $\tau_y$ as:

$$\tau_y \equiv (f_1 + f_2)r_y - (f_4 + f_3)r_y - \mu_2(f_1 + f_2 + f_3 + f_4)sgn(\dot{\theta}_y)r_y \tag{8}$$

Equation (8) can be substituted into Equation (7) to simplify a dynamic equation for the yaw joint as:

$$\tau_y = m_y \ddot{\theta}_y + c_y \dot{\theta}_y \tag{9}$$

Thus the dynamic equations for the yaw, pitch, and jaw joints are:

$$\begin{cases} \tau_y = m_y\,\ddot{\theta}_y + c_y\,\dot{\theta}_y \\ \tau_p = m_p\,\ddot{\theta}_p + c_p\,\dot{\theta}_p \\ \tau_j = m_p\,\ddot{\theta}_j + c_p\,\dot{\theta}_j \end{cases} \qquad (10)$$

Further, a set of equations to relate articulating torques to forces in the cables 236, 246, 256, 266 can be determined from Equations (4), (5), and (8) such that:

$$\begin{cases} \tau_y \equiv (f_1 + f_2)r_y - (f_4 + f_3)r_y - \mu_2(f_1 + f_2 + f_3 + f_4)sgn(\dot{\theta}_y)r_y \\ \tau_p \equiv (f_1 - f_2)r_p - \mu_1(f_1 + f_2)sgn(\dot{\theta}_a)r_p + (f_4 - f_3)r_p - \mu_1(f_3 + f_4)sgn(\dot{\theta}_b)r_p \\ \tau_j \equiv 2\left\{(f_1 - f_2)r_p - \mu_1(f_1 + f_2)sgn(\dot{\theta}_a)r_p - (f_4 - f_3)r_p + \mu_1(f_3 + f_4)sgn(\dot{\theta}_b)r_p\right\} \end{cases} \qquad (11)$$

This can be simplified by defining the following variables:

$$\begin{cases} \eta_{1a} \equiv \mu_1 sgn(\dot{\theta}_a) \\ \eta_{1b} \equiv \mu_1 sgn(\dot{\theta}_b) \\ \eta_2 \equiv \mu_2 sgn(\dot{\theta}_y) \end{cases} \qquad (12)$$

Such that Equations (11) can be expressed in matrix form as:

$$\begin{bmatrix} \tau_y \\ \tau_p \\ \tau_j \end{bmatrix} = \begin{bmatrix} r_y(1-\eta_2) & r_y(1-\eta_2) & -r_y(1+\eta_2) & -r_y(1+\eta_2) \\ r_p(1-\eta_{1a}) & -r_p(1+\eta_{1a}) & -r_p(1+\eta_{1b}) & r_p(1-\eta_{1b}) \\ 2r_p(1-\eta_{1a}) & -2r_p(1+\eta_{1a}) & 2r_p(1+\eta_{1b}) & -2r_p(1-\eta_{1b}) \end{bmatrix} \begin{bmatrix} f_1 \\ f_2 \\ f_3 \\ f_4 \end{bmatrix} \qquad (13)$$

An inverse kinematic model can be formed to determine desired yaw, pitch, and jaw angles of the end effector 270 to motor positions for each the motors of the IDU 13. As detailed above, movements in the yaw, pitch, and jaw DOFs of the end effector 270 can be achieved with the differential drive mechanism of the adapter 210. Further, movement in the negative of each of the DOF uses the same cables, e.g., cables 236, 246, 256, 266, but with the opposite direction or sign on each of the cables. It is noted that a negative sign on movement of a cable does not necessarily represent retraction of the cable but that the respective cable is kept under minimal tension to prevent the cable from going slack. Table 1, below, shows the combinations of movement in the positive direction in each of the DOFs. It is noted that translations of the jaw DOF are half due to each jaw "a, b" moving and contributing to the total jaw angle.

Table 1.

| Movement | Cable 236 | Cable 246 | Cable 256 | Cable 266 |
|----------|-----------|-----------|-----------|-----------|
| Yaw | $-s_y$ | $-s_y$ | $+s_y$ | $+s_y$ |
| Pitch | $+s_p$ | $-s_p$ | $-s_p$ | $+s_p$ |
| Jaw | $+0.5s_j$ | $-0.5s_j$ | $+0.5s_j$ | $-0.5s_j$ |

The movement of each of the cables in a linear combination of the cable movements in yaw, pitch, and jaw such that:

$$\begin{cases} s_1 = -s_y + s_p + 0.5s_j \\ s_2 = -s_y - s_p - 0.5s_j \\ s_3 = s_y - s_p + 0.5s_j \\ s_4 = s_y + s_p - 0.5s_j \end{cases} \tag{14}$$

Thus, the movement of each cable 236, 246, 256, 266 for each of the movements can be given by:

$$\begin{cases} s = r\theta \\ s \equiv cable\,translation\ (mm) \\ r \equiv pulley\ radius\ (mm) \\ \theta \equiv rotation\ of\ pully\ (rad) \end{cases} \tag{15}$$

Such that the displacement, e.g., translation, of each cable 236, 246, 256, 266 for movement in each of the DOFs is:

$$\begin{cases} s_y = r_y \theta_y \\ s_p = r_p \theta_p \\ s_j = r_j \theta_j \end{cases} \tag{16}$$

As described above, the IDU 13 includes a motor (not shown) that is associated with each of the motor interfaces 222, 224, 226, 228 to rotate a respective one of the drive screws 230, 240, 250, 260 which in turn retract or relax a corresponding cable 236, 246, 256, 266. The motors are rotated as a function of the desired pose of the end effector 270 from the following:

$$\begin{cases} s = \dfrac{k_p}{k_m} q \\ k_m \equiv gear\ ratio,\ i.e.\left(\dfrac{input-motor}{output-motor}\right), dimensionless \\ k_p \equiv screw\ pitch,\ i.e.\left(\dfrac{translation}{rotation}\right),\left(\dfrac{mm}{rad}\right) \\ q \equiv motor\ rotation\ (rad) \end{cases} \tag{17}$$

Noting that s is the overall translation of the respective cable such that a desired rotation of a motor can be shown as a function of the desired end effector pose as:

$$q_n = \frac{k_m}{k_p} s_n \qquad (18)$$

The desired rotation of each motor as a function of the desired end effector pose can be combined in a single matrix as:

$$\begin{bmatrix} q_1 \\ q_2 \\ q_3 \\ q_4 \end{bmatrix} = \frac{k_m}{k_p} \begin{bmatrix} -r_y & r_p & 0.5r_p \\ -r_y & -r_p & -0.5r_p \\ r_y & -r_p & 0.5r_p \\ r_y & r_p & -0.5r_p \end{bmatrix} \begin{bmatrix} \theta_y \\ \theta_p \\ \theta_j \end{bmatrix} \qquad (19)$$

If it is assumed that radii of each of the idlers 273, 275 and spindles 277, 279 are equal for each of the cables 236, 246, 256, 266, then Equation (19) can be simplified to:

$$\begin{bmatrix} q_1 \\ q_2 \\ q_3 \\ q_4 \end{bmatrix} = \frac{rk_m}{k_p} \begin{bmatrix} -1 & 1 & 0.5 \\ -1 & -1 & -0.5 \\ 1 & -1 & 0.5 \\ 1 & 1 & -0.5 \end{bmatrix} \begin{bmatrix} \theta_y \\ \theta_p \\ \theta_j \end{bmatrix} \qquad (20)$$

From the inverse kinematic model of Equation (20), a forward kinematic model can be obtained to relate motor position to the yaw, pitch, and jaw angles of the end effector 270. It is noted that Equation (20) has three unknowns and four equations. However, if proper tension is maintained in each of the cables 236, 246, 256, 266, Equation (20) can be solved in the reverse direction. For example, to solve for $\theta_j$, the first and third equations or the second and fourth equations can be added together. Since it is no more likely that either one of the sums is more accurate, an average of the two can be taken. Following this reasoning, a forward relationship can be expressed as follows:

$$\begin{bmatrix} \theta_y \\ \theta_p \\ \theta_j \end{bmatrix} = \frac{k_p}{4rk_m} \begin{bmatrix} -1 & -1 & 1 & 1 \\ 1 & -1 & -1 & 1 \\ 2 & -2 & 2 & -2 \end{bmatrix} \begin{bmatrix} q_1 \\ q_2 \\ q_3 \\ q_4 \end{bmatrix} \qquad (21)$$

It will be appreciated that Equation (21) can be obtained by taking a pseudoinverse, e.g., the Moore-Penrose inverse, of Equation (20).

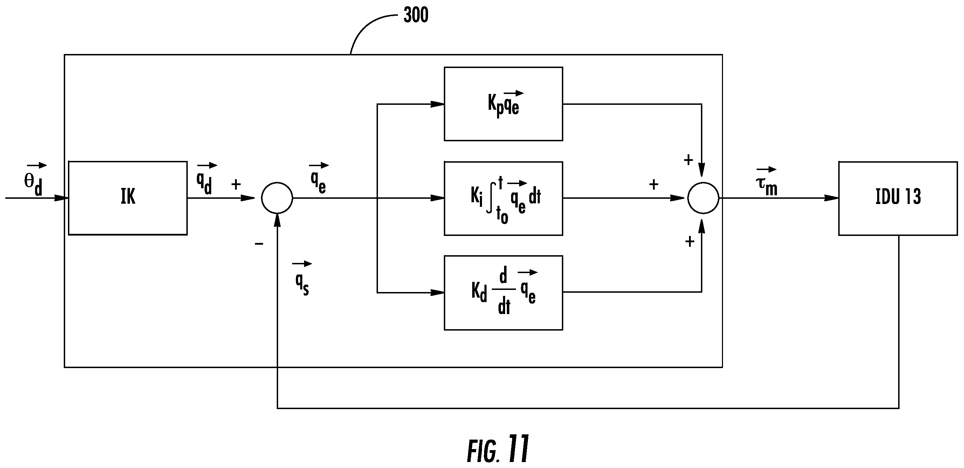

Referring to FIG. 11, the motors of the IDU 13 can be controlled by a proportional-integral-derivative (PID) controller 300 as detailed below in accordance with the present disclosure. The desired motor position $\vec{q_d}$ is calculated from a desired jaw angle $\vec{\theta_d}$ using Equation (19). The motor position is then sensed as $\vec{q_s}$ and subtracted from the desired position to generate a motor position error $\vec{q_e}$. The PID controller 300 then calculates a motor torque $\vec{\tau_m}$ which is outputted to the IDU 13. Propositional, integral, and derivative gains $K_p$, $K_i$, $K_d$ can be expressed in a control law as:

$$\vec{\tau}_m = K_p \vec{q}_e + K_i \int_{t_0}^{t} \vec{q}_e dt + K_d \frac{d}{dt} \vec{q}_e \qquad (22)$$

where $\vec{q_e}$ is calculated from Equation (20) as follows:

$$\vec{q}_e = \vec{q}_d - \frac{rk_m}{k_p} \begin{bmatrix} -1 & 1 & 0.5 \\ -1 & -1 & -0.5 \\ 1 & -1 & 0.5 \\ 1 & 1 & -0.5 \end{bmatrix} \vec{q}_s \qquad (23)$$

The PID controller 300 as shown in FIG. 11 may also be used to control stiffness in one or more DOF of the end effector 270. In addition, the PID controller 300 may calculate error in joint space instead of motor space as shown by utilizing the forward kinematics model of Equation (21).

As detailed above, the PID controller 300 alone does not directly or explicitly account for force within the cables 236, 246, 256, 266. For example, maintaining tension, e.g., preventing slack in the cables, is a requirement of the kinematics models detailed above. It will be appreciated that if the motors of the IDU 13 track the desired motor position with high precision and in sync with one another, that tension will be maintained in the cables 236, 246, 256, 266. In contrast, if the motors are unable to move to a desired motor position in sync with one or more of the other motors, then one or more of the cables 236, 246, 256, 266 may become slack. In embodiments, the cables 236, 246, 256, 266 may be pre-tensioned by a predetermined amount by monitoring current of the motors of the IDU 13 and/or monitoring torques sensors before the PID controller 300. By pre-tensioning the cables 236, 246, 256, 266, tension in the cables will be kept positive even if the motors of the IDU 13 move the cables 236, 246, 256, 266 out of sync.

In addition, the PID controller 300 alone does not maintain a clamping force in the jaws "a, b" when the jaws "a, b", are closed. For example, when the motors of the IDU 13 close the position loop according to Equation (20), the net clamping force is approximately zero. In embodiments, when the end effector 270 cannot reach a zero position, some closure force may remain when the jaws "a, b" are in the closed position. In embodiments, the jaw angle can be commanded to be negative such that closure force may remain when the jaws "a, b" are in the closed position. In such embodiments, a desired angle in the jaw DOF may be scaled and the inverse kinematic equation may be modified once the jaw angle is past zero such that the clamping force is modulated by changing a magnitude of the desired negative angle. However, this approach may cause confusion for a clinician as the end effector 270 may not open when it is commanded until the commanded jaw angle exceeds the desired negative angle. Further, there may be tracking errors in the jaw DOF due to the rescaling.

In accordance with embodiments of the robotic surgical system 1 (FIG. 1), the surgical robot 10 or processing unit 30 includes a null space (NS) controller 400 that is configured to provide cable tension and/or jaw clamping force. In such embodiments, the PID controller 300 is a primary positional controller and the NS controller 400 is a secondary controller.

The PID controller 300 continues to control a pose of the end effector 270 using Equation (20) as detailed above. As the PID controller 300 controls the pose of the end effector 270, another DOF, cable force, is created in the motor space from Equation (13) which can be related to the motor torque through the screws 230, 240, 250, 260. The cable force in each cable 236, 246, 256, 266 is related to torque of the respective motor as:

$$f_i = k_{ls}\tau_i, i = 1, 2, 3, 4 \qquad (24)$$

where $k_{ls}$ is a conversion coefficient that accounts for direction and efficiency of the respective screw 230, 240, 250, 260.

Assuming that the screws 230, 240, 250, 260 have the same radius, Equation (24) can be combined with Equation (13) such that:

$$\begin{bmatrix} \tau_y \\ \tau_p \\ \tau_j \end{bmatrix} = rk_{ls} \begin{bmatrix} (1-\eta_2) & (1-\eta_2) & -(1+\eta_2) & -(1+\eta_2) \\ (1-\eta_{1a}) & -(1+\eta_{1a}) & -(1+\eta_{1b}) & (1-\eta_{1b}) \\ 2(1-\eta_{1a}) & -2(1+\eta_{1a}) & 2(1+\eta_{1b}) & -2(1-\eta_{1b}) \end{bmatrix} \begin{bmatrix} \tau_1 \\ \tau_2 \\ \tau_3 \\ \tau_4 \end{bmatrix} \qquad (25)$$

which can be simplified to:

$$S \equiv rk_{ls} \begin{bmatrix} (1-\eta_2) & (1-\eta_2) & -(1+\eta_2) & -(1+\eta_2) \\ (1-\eta_{1a}) & -(1+\eta_{1a}) & -(1+\eta_{1b}) & (1-\eta_{1b}) \\ 2(1-\eta_{1a}) & -2(1+\eta_{1a}) & 2(1+\eta_{1b}) & -2(1-\eta_{1b}) \end{bmatrix} \qquad (26)$$

As shown above, Equation 25 is under constrained such that for the same articulating torque, there may be different ways to generate the motor torque to balance Equation (25). For example, motor torques $\vec{\tau}_{motor}$ may satisfy Equation (25) and the requesting articulating joint torques $\vec{\tau}_{joint}$ such that:

$$\vec{\tau}_{joint} = S\vec{\tau}_{motor} \qquad (27)$$

where the motor torques $\vec{\tau}_{motor}$ could be torques that are generated from the PID controller 300 as shown above with respect to Equation (22). As Equation (25), and thus the matrix S of Equation (26), is unconstrained, additional motor torques $\vec{\Delta\tau}_{motor}$ may be added to the right side of Equation (27) such that:

$$\vec{\tau}_{jo\text{int}} = S\left(\vec{\tau}_{motor} + \vec{\Delta\tau}_{motor}\right) \tag{28}$$

This is satisfied by constraining the additional motor torques $\vec{\Delta\tau}_{motor}$ within null space of matrix S as:

$$\vec{0} = S\vec{\Delta\tau}_{motor} \tag{29}$$

such that there is no net change in the articulating joint torques $\vec{\Delta\tau}_{joint}$ determined by the PID controller 300.

From the above, it is possible to develop the secondary NS controller 400 to adjust the motor torques $\vec{\Delta\tau}_{motor}$ generated by the PID controller 300 such that the NS controller 400 cascades after the PID controller 300 to directly adjust the motor torques $\vec{\Delta\tau}_{motor}$. The motor torques $\vec{\Delta\tau}_{motor}$ may be measured directly by additional torque sensors that detect the output torque of the motors of the IDU 13. For a detailed description of suitable torque sensors, reference can be made to U.S. Patent No. 9,987094.

First, maintaining tension in the cables 236, 246, 256, 266 will be addressed in accordance with the present disclosure. During normal operation the end effector 270 has three DOF with the articulating torques defined in Equation (13). These torques can be adjusted without impacting the position of the end effector 270 as long as the additional motor torques $\vec{\Delta\tau}_{motor}$ satisfy Equation (29) such that vectors in null space can be expressed as:

$$\vec{\Delta\tau}_{motor} = (I_{4\times4} - S^T(SS^T)^{-1}S)\vec{z} \tag{30}$$

where matrix I is a 4x4 identity matrix; $S^T$ is the transpose of matrix S, and $\vec{z}$ is an arbitrary vector with a dimension of 4. The arbitrary vector $\vec{z}$ can be projected or decomposed through a helper matrix by:

$$N = (I_{4\times4} - S^T(SS^T)^{-1}S) \tag{31}$$

Then, substituting Equation (26) into Equation (31) and setting the friction coefficients $\mu_1$ and $\mu_2$ to zero produces:

$$N = \begin{bmatrix} 0.25 & 0.25 & 0.25 & 0.25 \\ 0.25 & 0.25 & 0.25 & 0.25 \\ 0.25 & 0.25 & 0.25 & 0.25 \\ 0.25 & 0.25 & 0.25 & 0.25 \end{bmatrix} \tag{32}$$

Next, the influence of the constant $rK_{ls}$ is extracted from the null space defined in Equation (29) by assuming that the arbitrary vector $\vec{z}$ has the same unit of measure as the motor torques $\vec{\tau}_{motor}$. By substituting Equation (32) into Equation (30):

$$\vec{\Delta\tau}_{motor} = \begin{bmatrix} 0.25 & 0.25 & 0.25 & 0.25 \\ 0.25 & 0.25 & 0.25 & 0.25 \\ 0.25 & 0.25 & 0.25 & 0.25 \\ 0.25 & 0.25 & 0.25 & 0.25 \end{bmatrix} \begin{bmatrix} z_1 \\ z_2 \\ z_3 \\ z_4 \end{bmatrix} \tag{33}$$

which is rearranged as:

$$\begin{bmatrix} \Delta\tau_{motor,1} \\ \Delta\tau_{motor,2} \\ \Delta\tau_{motor,3} \\ \Delta\tau_{motor,4} \end{bmatrix} = 0.25 \begin{bmatrix} z_1 + z_2 + z_3 + z_4 \\ z_1 + z_2 + z_3 + z_4 \\ z_1 + z_2 + z_3 + z_4 \\ z_1 + z_2 + z_3 + z_4 \end{bmatrix} \tag{34}$$

where the additional motor torque vector $\overrightarrow{\Delta\tau}_{motor}$ is explicitly expressed by its elements as:

$$\overrightarrow{\Delta\tau}_{motor} = \begin{bmatrix} \Delta\tau_{motor,1} \\ \Delta\tau_{motor,2} \\ \Delta\tau_{motor,3} \\ \Delta\tau_{motor,4} \end{bmatrix} \tag{35}$$

and since arbitrary vector $\vec{z}$ is an arbitrary vector, another arbitrary scalar $\Delta\tau$ can be defined as:

$$\Delta\tau = 0.25(z_1 + z_2 + z_3 + z_4) \tag{36}$$

which results in:

$$\begin{bmatrix} \Delta\tau_{motor,1} \\ \Delta\tau_{motor,2} \\ \Delta\tau_{motor,3} \\ \Delta\tau_{motor,4} \end{bmatrix} = \begin{bmatrix} \Delta\tau \\ \Delta\tau \\ \Delta\tau \\ \Delta\tau \end{bmatrix} \tag{37}$$

As shown by inserting Equation (37) into Equation (28), it is clear that the same torque can be added to each motor of the IDU 13 without changing the articulating torques for the yaw, pitch, and jaw DOFs. Thus, if the additional motor torque for each motor is the same, a position determined by the PID controller 300 will not be impacted. As detailed above with respect to Equation (25) this assumes that the friction is ignored which is allowed as the additional torque of each motor does not articulate the joints but generates internal forces within the system and contributes to a stiffness of the system.

However, when the friction coefficients $\mu_1$ and $\mu_2$ are significant, the matrix S is solved symbolically such that the null space of Equation (29) is expressed as:

$$\overrightarrow{\Delta\tau}_{motor} = \begin{bmatrix} \dfrac{(1+\eta_{1a})(1+\eta_2)}{(1+\eta_{1b})(1-\eta_2)} \\[2ex] \dfrac{(1-\eta_{1a})(1+\eta_2)}{(1+\eta_{1b})(1-\eta_2)} \\[2ex] \dfrac{1-\eta_{1b}}{1+\eta_{1b}} \\[2ex] 1 \end{bmatrix} \Delta\tau \tag{38}$$

It is noted that if the friction coefficients $\mu_1$ and $\mu_2$ are zero, Equation (38) degrades to Equation (37) such that Equation (37) is a first order approximation of Equation (38). Thus, Equation (38) provides relationships of the additional motor torques $\overrightarrow{\Delta\tau}_{motor}$ that can be adjusted without impacting the articulating torques in jaw, pitch, and jaw DOFs to avoid impacting the position determined by the PID controller 300.

As Equation (38) is flexible in the implementation of the NS controller 400, several methods may be used to adjust the torque for each motor of the IDU in view of a measured torque $\vec{\tau s}$.

In a first method of maintaining cable tension, torque is adjusted at each motor according to a measured torque $\vec{\tau_s}$ which can be taken from a measured current or from a physical torque sensor as detailed above. In practice, when the NS controller 400 is operating in the first method, positional gain of the PID controller 300 as shown in Equation (22) is necessarily high. Further, to maintain minimum tension $f_{min}$ in the cables 236, 246, 256, 266, two of the four motors of the IDU 13 keep minimum tensions in the respective cables while the other two motors of the IDU 13 take over a workload from the two motors maintaining the minimum tension $f_{min}$ in addition to the expected workload. This is reflected in Table 1, above, which requires the positional change be distributed to the motor pairs with different signs by the same amounts such that each pair of motors of the IDU 13 that are physical connected by a pair of cables, e.g., first and second cables 236, 246 and third and fourth cables 256, 266.

The differential drive mechanism detailed above is a push-pull mechanism with two pairs of motors. However, in the first method the pusher does not actively push but maintains the minimum tension and the puller motor pulls the pusher motor to the desired position such that the puller motor of each pair closes the positional loop for both motors of the pair which requires the proportional gains to be high and the maximum current of the puller motor to be increased significantly. Even in view of the increased gains, the first method is extremely good at maintaining the minimum tension $f_{min}$ in each cable.

To keep the tension of each cable 236, 246, 256, 266 at a minimum tension $f_{min}$ as the motors of the IDU 13 articulate the end effector 270, a minimum $\Delta\tau$ is determined as:

$$
\begin{bmatrix}
\dfrac{(1+\eta_{1a})(1+\eta_2)}{(1+\eta_{1b})(1-\eta_2)}\Delta\tau + \tau_{s,1} \\[2mm]
\dfrac{(1-\eta_{1a})(1+\eta_2)}{(1+\eta_{1b})(1-\eta_2)}\Delta\tau + \tau_{s,2} \\[2mm]
\dfrac{1-\eta_{1b}}{1+\eta_{1b}}\Delta\tau + \tau_{s,3} \\[2mm]
\Delta\tau + \tau_{s,4}
\end{bmatrix}
\geq \frac{1}{k_{ls}}
\begin{bmatrix}
f_{min} \\ f_{min} \\ f_{min} \\ f_{min}
\end{bmatrix}
\tag{39}
$$

If the adjustment torque $\Delta\tau$ is the minimum torque that satisfies Equation (29), the adjustment torque $\Delta\tau$ from the NS controller 400 is expressed as:

$$
\vec{\tau}_{null} =
\begin{bmatrix}
\dfrac{(1+\eta_{1a})(1+\eta_2)}{(1+\eta_{1b})(1-\eta_2)}\Delta\tau \\[2mm]
\dfrac{(1-\eta_{1a})(1+\eta_2)}{(1+\eta_{1b})(1-\eta_2)}\Delta\tau \\[2mm]
\dfrac{1-\eta_{1b}}{1+\eta_{1b}}\Delta\tau \\[2mm]
\Delta\tau
\end{bmatrix}
\tag{40}
$$

In this first method, torque is adjusted at each individual motor of the IDU 13. The measured torque $\vec{\tau_s}$ is checked and adjusted to ensure that the tension in each of the cables 236, 246, 256, 266 is greater than the minimum tension $f_{min}$.

In a second method of maintaining cable tension, the physical connection between each pair of cables, e.g., first and second cable 236, 246 and third and fourth cable 256, 266, are considered such that the constraints defined in Equation (39) are relaxed. Specifically, instead of verifying torque of each motor, the torque of each pair of motors of the IDU 13 is constrained by an average of each motor group or pair of motors of the IDU 13.

If the average of the measured torque $\vec{\tau_s}$ meets the requirements as detailed below, then the puller motor of each pair is working harder than the pusher motor but since there is still a net torque in each of the motors of the pair, e.g., the pusher and puller, it means that the cable of the pusher motor is also likely under tension. However, as each motor is not individually checked, the second method may not provide as consistent result in maintaining tension in each of the cables greater than the minimum tension $f_{min}$. However, as the puller motor of each pair of motors is not required to take the entire load of the pusher motor of each pair of motors such that the load on the motors of the IDU 13, and the stiffness of the joint, can be reduced.

In the second method, the average of each of the motor groups is expressed as:

$$\begin{bmatrix} \dfrac{(1+\eta_{1a})(1+\eta_2)}{(1+\eta_{1b})(1-\eta_2)}\Delta\tau+\tau_{s,1}+\dfrac{(1-\eta_{1a})(1+\eta_2)}{(1+\eta_{1b})(1-\eta_2)}\Delta\tau+\tau_{s,2} \\[2ex] \dfrac{1-\eta_{1b}}{1+\eta_{1b}}\Delta\tau+\tau_{s,3}+\Delta\tau+\tau_{s,4} \end{bmatrix} \geq \dfrac{1}{2k_{ls}}\begin{bmatrix} f_{min} \\ f_{min} \end{bmatrix} \qquad (41)$$

Similar to the first method, the minimum adjustment torque $\Delta\tau$ is determined that fulfills Equation (41) such that output from the NS controller 400 takes the form of Equation (40). It is not necessary to show that if Equation (39) holds that Equation (41) will also hold. However, it is also clear that Equation (41) provides a less certain constraint that the minimum tension $f_{min}$ of each cable is maintained. However, as detailed above, as a result of each pair of cables being coupled together, as long as the puller motor of each pair of motors has a measured torque $\vec{\tau}_s$ greater than the average, then it is likely that the pusher motor is also maintaining the minimum tension $f_{min}$ in its related cable. Further, it is noted that Equation (41) accounts for an influence of friction in the calculation of the adjustment torque $\Delta\tau$.

In a third method of maintaining cable tension, the measured torque $\vec{\tau}_s$ of each motor of the IDU 13 is tracked and maintained above a targeted minimum torque $\tau_{min}$ based on a measured torque $\vec{\tau}_s$ that corresponds to the minimum tension $f_{min}$ desired in each of the cables 236, 246, 256, 266. The third method has a simple controller in the form of:

$$\Delta\tau = \tau_{min} + K(\tau_{min} - \tau_{s,min}) \qquad (42)$$

where $\tau_{min}$ is the targeted a minimum torque; $\tau_{s,\,min}$ is a sensed minimum torque, and K is a gain factor.

The adjustment torque $\Delta\tau$ in Equation (42) can provide varying degrees of certainty of maintaining tension in the cables 236, 246, 256, 266. For example, when the gain factor K=1, the adjustment torque $\Delta\tau$ will adjust every motor torque $\vec{\tau}_{motor}$ by the minimum torque $\tau_{s,min}$ such that each motor torque $\vec{\tau}_{motor}$ will be at or above the targeted minimum torque $\tau_{min}$ to maintain the minimum tension $f_{min}$ in the cables 236, 246, 256, 266. This will have a similar result as the first method detailed above where the puller motor of each pair of motors will take on the entire workload of the respective pusher motor while providing certainty that the minimum tension $f_{min}$ is maintained. When the gain factor K<1, certainty of maintaining the minimum tension $f_{min}$ is reduced while the additional workload on the puller motor of each pair of motors is reduced. In an extreme case, when the gain factor K=0, a constant is added to each motor torque $\vec{\tau}_{motor}$ to maintain the minimum tension $f_{min}$.

The third method allows for consideration of friction from Equation (38) by scaling the four elements of Equation (38) to account for friction and to make the highest adjustment torque $\Delta\tau$ equal to the output of Equation (42).

The third method allows for allowing for tuning of the gain factor K to account for performance requirements of other controllers such as an overall power, a maximum current that the motors of the IDU 13 can provide, and/or a stiffness of the j oint. This allows for the gain factor $K$ to be increased when certainty of maintaining cable tension is critical and workload on the motors of the IDU 13 can be increased and to be reduced when certainty of maintaining cable tension is less critical and workload on the motors of the IDU 13 needs to be reduced.

Second, generating a clamping force between jaws "a, b" will be addressed in accordance with the present disclosure. It may be beneficial to adjust the clamping force between jaws "a, b" depending on the type of instrument of the end effector 270. For example, when the jaws "a, b" form a needle driver, a high clamping force is required and when the jaws "a, b" are a bowel grasper a much lower clamping force is required.

In a first method or technique of generating clamping force, the clamping force is generated by over clamping the jaws, e.g., commanding the desired jaw angle $\vec{\theta}_d$ to a negative value. In the first technique, an amount of clamping force generated depends on the overall stiffness of the PID controller 300 and a magnitude of the desired negative angle. To maintain cable tension, Equation (20) is modified after the actual jaw angle passes zero as follows:

$$\begin{bmatrix} q_1 \\ q_2 \\ q_3 \\ q_4 \end{bmatrix} = \frac{rk_m}{k_p}\begin{bmatrix} -1 & 1 & 0 \\ -1 & -1 & -0.5 \\ 1 & -1 & 0 \\ 1 & 1 & -0.5 \end{bmatrix}\begin{bmatrix} \theta_y \\ \theta_p \\ \theta_j \end{bmatrix} \qquad (43)$$

By modifying Equation (20), the puller motors of each pair of motors continue to pull while the pusher motors are prevented from relaxing.

As a commanded jaw angle $\theta_j$ does not go negative, the commanded jaw angle $\theta_j$ must be remapped in the first technique. As detailed above, if the commanded jaw angle $\theta_j$ is remapped linearly, a clinician may notice a significant difference between a commanded jaw angle $\theta_j$ and an actual jaw angle. To improve a clinician's experience and more closely track the commanded jaw angle $\theta_j$ with the actual jaw angle, the commanded jaw angle $\theta_j$ can be mapped to the actual jaw angle nonlinearly. The commanded jaw angle $\theta_j$ can be mapped using different slopes where for most of a range of the commanded jaw angle $\theta_j$ the slope is substantially linear and as the commanded jaw angle $\theta_j$ approaches zero, a steeper slope is used. For example, a logarithmic function may be used such that when the commanded jaw angle $\theta_j \in$ [0,10] can be mapped to [-10, 10] roughly through $6\log_4(\theta_j + 0.1)$. When the commanded jaw angle $\theta_j$ changes from 0.9 to 10, the mapped angle $\theta_m$ changes from 0 to 10 such that the mapping is substantially linear. In contrast, when the commanded jaw angle $\theta_j$ changes from 0 to 0.9, the mapped angle $\theta_m$ changes from -10 to 0 such that the slope is significantly non-linear and steep.

In a second method or technique for generating a clamping force between jaws "a, b", the NS controller 400 can be used to generate the clamping force when the jaws "a, b" are in closed position. When the jaws "a, b" are in the closed position, the end effector 270 has two DOF such that Equation (11) degrades, as the articulating torque $\tau_j$ of in the jaw DOF is now zero, to the following:

$$\begin{cases} \tau_y \equiv (f_1 + f_2)r_y - (f_4 + f_3)r_y - \mu_2(f_1 + f_2 + f_3 + f_4)sgn(\dot{\theta}_y)r_y \\ \tau_p \equiv (f_1 - f_2)r_p - \mu_1(f_1 + f_2)sgn(\dot{\theta}_p)r_p + (f_4 - f_3)r_p - \mu_1(f_3 + f_4)sgn(\dot{\theta}_p)r_p \end{cases} \qquad (44)$$

Two new variables can be defined as follows:

$$\begin{cases} \eta_1 = \mu_1 sgn(\dot{\theta}_p) \\ \eta_2 = \mu_2 sgn(\dot{\theta}_y) \end{cases} \qquad (45)$$

to simplify Equation (44) to:

$$\begin{bmatrix} \tau_y \\ \tau_p \end{bmatrix} = rk_{ls} \begin{bmatrix} (1-\eta_2) & (1-\eta_2) & -(1+\eta_2) & -(1+\eta_2) \\ (1-\eta_1) & -(1+\eta_1) & -(1+\eta_1) & (1-\eta_1) \end{bmatrix} \begin{bmatrix} \tau_1 \\ \tau_2 \\ \tau_3 \\ \tau_4 \end{bmatrix} \qquad (46)$$

Similarly, Equation (26) also degrades to:

$$S = rk_{ls} \begin{bmatrix} (1-\eta_2) & (1-\eta_2) & -(1+\eta_2) & -(1+\eta_2) \\ (1-\eta_1) & -(1+\eta_1) & -(1+\eta_1) & (1-\eta_1) \end{bmatrix} \qquad (47)$$

which can be expressed for null space of matrixes $S_1$ to $S_4$ as defined in Equation (47) as:

$$\Delta\vec{\tau}_{motor} = \begin{bmatrix} \dfrac{1+\eta_1}{1-\eta_2} \\ \dfrac{\eta_2-\eta_1}{1-\eta_2} \\ 1 \\ 0 \end{bmatrix} \Delta\tau_3 + \begin{bmatrix} \dfrac{\eta_2+\eta_1}{1-\eta_2} \\ \dfrac{1-\eta_1}{1-\eta_2} \\ 0 \\ 1 \end{bmatrix} \Delta\tau_4 \qquad (48)$$

where $\Delta\tau_3$ and $\Delta\tau_4$ are arbitrary scalars.

By setting the friction coefficients $\mu_1$ and $\mu_2$ to zero it can be shown that the additional motor torques $\overrightarrow{\Delta\tau}_{motor}$ for motors 2 and 4 are equal and that the additional motor torques $\overrightarrow{\Delta\tau}_{motor}$ for motors 1 and 3 are equal:

$$\begin{bmatrix} \Delta\tau_{motor,1} \\ \Delta\tau_{motor,2} \\ \Delta\tau_{motor,3} \\ \Delta\tau_{motor,4} \end{bmatrix} = \begin{bmatrix} \Delta\tau_3 \\ \Delta\tau_4 \\ \Delta\tau_3 \\ \Delta\tau_4 \end{bmatrix} \tag{49}$$

Thus, to balance internal forces in the end effector 270:

$$f_{clamp} = f_4 - f_3 = f_2 - f_1 \tag{50}$$

which constrains the additional motor torques $\overrightarrow{\Delta\tau}_{motor}$ to:

$$f_{clamp} = k_{ls}\Delta\tau_{clamp} = f_2 - f_1 \tag{51}$$

$$f_{clamp} = k_{ls}\Delta\tau_{clamp} = k_{ls}(\Delta\tau_{motor,4} - \Delta\tau_{motor,3}) = k_{ls}(\Delta\tau_{motor,2} - \Delta\tau_{motor,1}) \tag{52}$$

Equation (52) illustrates that the difference between $\Delta\tau_3$ and $\Delta\tau_4$ can be used to adjust the clamping force $\Delta\tau_{clamp}$ between jaws "a, b" which is defined as:

$$\Delta\tau_{clamp} = \Delta\tau_4 - \Delta\tau_3 \tag{53}$$

Such that Equation (49) can be rewritten as:

$$\begin{bmatrix} \Delta\tau_{motor,1} \\ \Delta\tau_{motor,2} \\ \Delta\tau_{motor,3} \\ \Delta\tau_{motor,4} \end{bmatrix} = \begin{bmatrix} \Delta\tau_3 \\ \Delta\tau_3 + \Delta\tau_{clamp} \\ \Delta\tau_3 \\ \Delta\tau_3 + \Delta\tau_{clamp} \end{bmatrix} \tag{54}$$

When Equation (54) is compared to Equation (37) it is clear that cable tension can be maintained in the first, second, and third methods while adjusting the clamping force $\Delta\tau_{clamp}$ between jaws "a, b" once the jaws "a, b" are in the closed position.

When the friction coefficients $\mu_1$ and $\mu_2$ are included, the clamping force $\Delta\tau_{clamp}$ of Equation (50) depends on the sign in Equation (45). For example, when $sgn(\dot{\theta}_p)$ is positive, the second part of Equation (44) is rearranged as:

$$\tau_p = r_p \left\{ (f_4 - f_3) - \mu_1(f_3 + f_4) - \left( (f_2 - f_1) + \mu_1(f_1 + f_2) \right) \right\} \tag{55}$$

In this example, the articulating torque for pitch $\tau_p$ is provided by the fourth cable 266. To provide the articulating torque for pitch $\tau_p$, the frictional forces that must be overcome by the fourth cable 266 are proportional to the total force of the third and fourth cables 256, 266 and represented by $\mu_1(f_3 + f_4)$. In addition, the fourth cable 266 must also overcome a countering force provided by the opposite jaw which is shown as $f_2 - f_1$. Further, the fourth cable 266 must also overcome the friction of the other jaw represented as $\mu_1(f_1 + f_2)$. The internal torque or clamping force $\Delta\tau_{clamp}$ can be expressed as:

$$\Delta\tau_{clamp} = r_p \left( f_2 - f_1 + \mu_1(f_1 + f_2) \right) \tag{56}$$

Further, the motor torques $\overrightarrow{\Delta\tau}_{motor}$ can absorbed the coefficient $k_{ls}$ as:

$$\Delta\tau_{clamp} = \Delta\tau_{motor,2} - \Delta\tau_{motor,1} + \mu_1\left(\Delta\tau_{motor,1} + \Delta\tau_{motor,2}\right) \quad\quad (57)$$

To maintain the clamping force $\Delta\tau_{clamp}$ between jaws "a, b", Equation (48) can be expressed as:

$$\vec{\Delta\tau}_{motor} = \begin{bmatrix} \dfrac{1+\mu_1}{1-\eta_2}\Delta\tau_3 + \dfrac{\eta_2+\mu_1}{1-\eta_2}\Delta\tau_4 \\[2mm] \dfrac{\eta_2-\mu_1}{1-\eta_2}\Delta\tau_3 + \dfrac{1-\mu_1}{1-\eta_2}\Delta\tau_4 \\[2mm] \Delta\tau_3 \\[1mm] \Delta\tau_4 \end{bmatrix} \quad\quad (58)$$

Substituting Equation (57) into Equation (58) in which $\Delta\tau_4$ is expressed as a function of $\Delta\tau_3$ and the clamping force $\Delta\tau_{clamp}$ yields:

$$\vec{\Delta\tau}_{motor} = \begin{bmatrix} \dfrac{(\mu_1+\eta_2)\Delta\tau_{clamp} + (1+\eta_2)(1+\mu_1)\Delta\tau_3}{(1-\eta_2)(1-\mu_1)} \\[3mm] \dfrac{\Delta\tau_{clamp} + (1+\eta_2)\Delta\tau_3}{1-\eta_2} \\[3mm] \Delta\tau_3 \\[2mm] \dfrac{\Delta\tau_{clamp} + (1+\mu_1)\Delta\tau_3}{1-\mu_1} \end{bmatrix} \quad\quad (59)$$

In a similar manner, when $sgn(\dot{\theta}_p)$ is negative, the second part of Equation (44) can be rearranges as:

$$\tau_p = -r_p\left\{(f_2-f_1) - \mu_1(f_1+f_2) - \left((f_4-f_3) + \mu_1(f_3+f_4)\right)\right\} \quad\quad (60)$$

In this example, the articulating torque for pitch is provided by the second cable 246 such that the internal torque or clamping force $\Delta\tau_{clamp}$ can be expressed as:

$$\Delta\tau_{clamp} = \Delta\tau_{motor,4} - \Delta\tau_{motor,3} + \mu_1\left(\Delta\tau_{motor,3} + \Delta\tau_{motor,4}\right) \quad\quad (61)$$

To maintain the clamping force $\Delta\tau_{clamp}$ between jaws "a, b", Equation (48) can be expressed as:

$$\vec{\Delta\tau}_{motor} = \begin{bmatrix} \dfrac{1-\mu_1}{1-\eta_2}\Delta\tau_3 + \dfrac{\eta_2-\mu_1}{1-\eta_2}\Delta\tau_4 \\[2mm] \dfrac{\eta_2+\mu_1}{1-\eta_2}\Delta\tau_3 + \dfrac{1+\mu_1}{1-\eta_2}\Delta\tau_4 \\[2mm] \Delta\tau_3 \\[1mm] \Delta\tau_4 \end{bmatrix} \quad\quad (62)$$

Substituting Equation (61) into Equation (61) provides:

$$\vec{\Delta \tau}_{motor} = \begin{bmatrix} \dfrac{(-\mu_1 + \eta_2)\Delta\tau_{clamp} + (1+\eta_2)(1-\mu_1)\Delta\tau_3}{(1-\eta_2)(1+\mu_1)} \\[2em] \dfrac{\Delta\tau_{clamp} + (1+\eta_2)\Delta\tau_3}{1-\eta_2} \\[2em] \Delta\tau_3 \\[1em] \dfrac{\Delta\tau_{clamp} + (1-\mu_1)\Delta\tau_3}{1+\mu_1} \end{bmatrix} \tag{63}$$

A governing equation can be developed in view of the symmetry between Equations (59) and (63) as:

$$\begin{bmatrix} \Delta\tau_{motor,1} \\ \Delta\tau_{motor,2} \\ \Delta\tau_{motor,3} \\ \Delta\tau_{motor,4} \end{bmatrix} = \begin{bmatrix} \dfrac{(\eta_1 + \eta_2)\Delta\tau_{clamp} + (1+\eta_2)(1+\eta_1)\Delta\tau_3}{(1-\eta_2)(1-\eta_1)} \\[2em] \dfrac{\Delta\tau_{clamp} + (1+\eta_2)\Delta\tau_3}{1-\eta_2} \\[2em] \Delta\tau_3 \\[1em] \dfrac{\Delta\tau_{clamp} + (1+\eta_1)\Delta\tau_3}{1-\eta_1} \end{bmatrix} \tag{64}$$

which has two independent variables, the clamping force $\Delta\tau_{clamp}$ and $\Delta\tau_3$. The clamping force $\Delta\tau_{clamp}$ can be used to control an amount of force between jaws "a, b" such that Equation (64) provides a direct means for managing a clamping torque and force in the jaw DOF. Further, the free variable $\Delta\tau_3$ can be used to maintain cable tension as detailed above.

Equation (64) depends on the assumption that the jaws "a, b" of the end effector 270 are closed such that the DOF of the end effector 270 are reduced to two such that Equation (44) holds. In use, this condition can be evaluated through the forward kinematic model of Equation (21). To verify the forward kinematic model of Equation (21) it may be advantageous to use the motor positions provided by encoders for the motors of the IDU 13 due to the physical separation of the motors and the end effector 270. As the motor encoders will induce some uncertainty, a clamping threshold can be set such that once the calculated jaw angle is smaller than the clamping threshold, the clamping torque adjustment as noted in Equation (64) can be applied gradually over a short period of time, e.g., 0.5 seconds. Further, as the adjustments are proportional to the two free variables, the clamping force $\Delta\tau_{clamp}$ and $\Delta\tau_3$, if the adjustments are increased proportionally, the position controlled by the PID controller 300 may be impacted.

As the clamping force is only provided on instruction from a clinician interfacing with the robotic surgical system 1 (FIG. 1), when a clinician signals an intent to open the jaws "a, b" of the end effector 270 the clamping force may be released faster than the addition of clamping force, e.g., 0.1 seconds. As the clinician can visually observe the jaws "a, b" opening once the intent is clear, a desired jaw angle $\vec{\theta_d}$ can be used to determine a releasing threshold instead of a calculated jaw angle as detailed above. Once the desired jaw angle $\vec{\theta_d}$ crosses the clamping threshold, the clamping torque in the form of the clamping force $\Delta\tau_{clamp}$ can be quickly applied. In addition, a hysteresis can be built between the clamping threshold and the releasing threshold to avoid a constant crossing between applying a clamping force and releasing a clamping force.

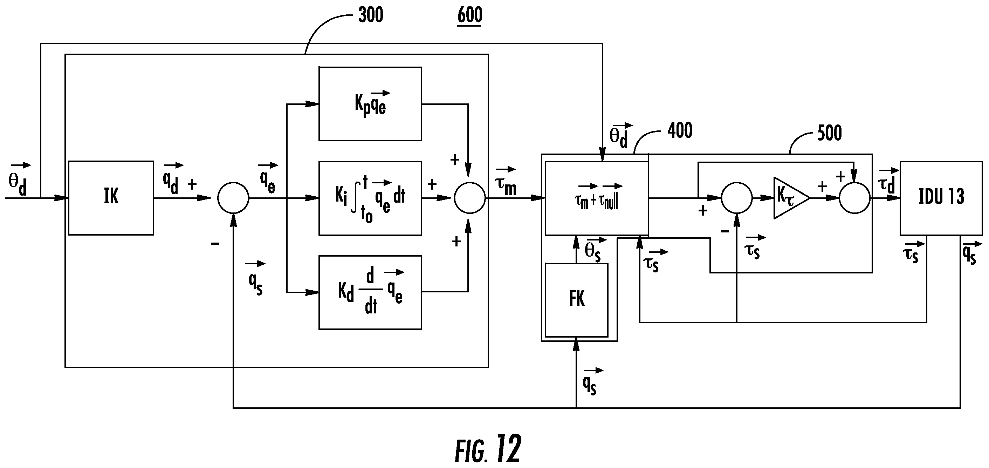

Referring now to FIG. 12, a total controller 600 that combines the PID controller 300, the NS controller 400, and a combination controller 500 provided in accordance with the present disclosure to control the position of the end effector 270 in yaw, pitch, and jaw and to maintain cable tension and generate a clamping force when needed. The PID controller 300 receives the desired jaw angle $\vec{\theta_d}$ and outputs motor torques $\vec{\tau}_m$ to the NS controller 400. In addition, the PID controller 300 receives the position of the motors $\vec{q_s}$ of the IDU 13 as part of a feedback loop.

The NS controller 400 receives the motor torques $\vec{\tau}_m$ from the PID controller 300 and the desired jaw angle $\vec{\theta_d}$. The NS controller 400 can utilize any of the methods for maintaining cable tension and/or techniques for generating a clamping force as detailed above. The desired jaw angle $\vec{\theta_d}$ can be used to calculate a desired angular velocity about each joint and to pick a corresponding sign for the friction coefficients $\mu_1$ and $\mu_2$, e.g., opposite the direction of desired angular velocity. Alternatively, friction may be ignored to simplify the NS controller 400. The NS controller 400 also receives the position of the motors $\vec{q_s}$ of the IDU 13 as part of a feedback loop and uses the forward kinematics model to generate calculated joint angles $\vec{\theta_s}$ to detect when the system loses a DOF, e.g., when the calculated joint angles $\vec{\theta_s}$ are less than a clamping threshold, to determine when to apply a clamping torque. In addition, the NS controller 400 may also receive a sensed

torque $\vec{\tau}_s$ to maintain cable tension.

The NS controller 400 outputs the motor torques $\vec{\tau}_m$ and null torques $\vec{\tau}_{null}$ to the combination controller 500. The combination controller 500 adds the null torques $\vec{\tau}_{null}$ to the motor torques $\vec{\tau}_m$ to calculate a desired torque $\vec{\tau}_d$ from a sum of the motor torques $\vec{\tau}_m$ and null torques $\vec{\tau}_{null}$ for output to the IDU 13. In embodiments where a sensed torque $\vec{\tau}_s$ is available, the combination controller 500 receives the sensed torque $\vec{\tau}_s$ and closes the desired combination controller 500 then outputs the desired torque $\vec{\tau}_d$. The proportional gain $K_s$ is implemented to reduce error between the desired torque $\vec{\tau}_d$ and the sensed torque $\vec{\tau}_s$. Specifically, a sum of the motor torques $\vec{\tau}_m$ and null torques $\vec{\tau}_{null}$ may be the desired torque $\vec{\tau}_d$ and the proportional gain $K_s$ is implemented such that the sensed torque $\vec{\tau}_s$ approaches the sum of the motor torques $\vec{\tau}_m$ and null torques $\vec{\tau}_{null}$. Thus, when an error between the desired torque $\vec{\tau}_d$ and the sensed torque $\vec{\tau}_s$ is small, the proportional gain $K_s$ is also small. As detailed herein, the combination controller 500 is a tertiary controller.

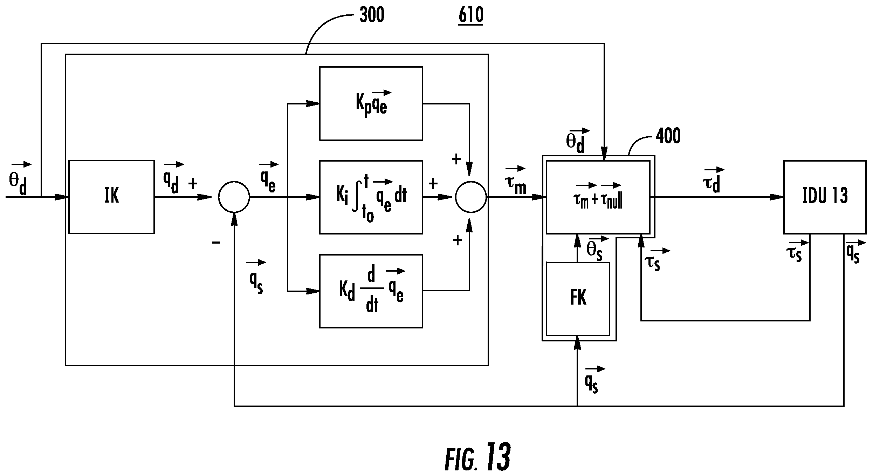

Referring to FIG. 13, another total controller 610 that combines the PID controller 300 and the NS controller 400 is disclosed in accordance with the present disclosure to control the position of the end effector 270 in yaw, pitch, and jaw and to maintain cable tension. As shown, the NS controller 400 adds the motor torques $\vec{\tau}_m$ and the null torques $\vec{\tau}_{null}$ and outputs the desired torque $\vec{\tau}_d$ directly to the IDU 13. The total controller 610 may be used when the NS controller 400 maintains cable tension but does not generate a clamping force.

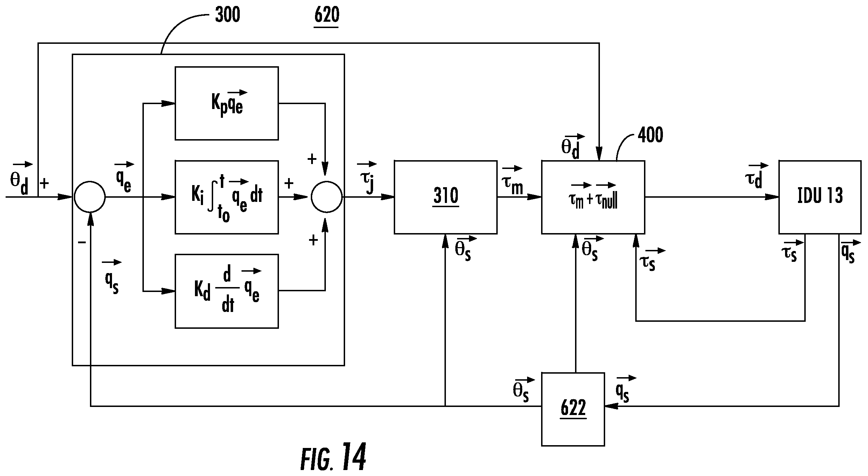

With reference to FIG. 14, another total controller 620 that combines the PID controller 300 and the NS controller 400 is disclosed in accordance with the present disclosure to control the position of the end effector 270 in yaw, pitch, and jaw and to maintain cable tension and generate a clamping force when needed. The total controller 620 also includes a joint space converter 622 that converts the motor positions $\vec{q}_s$ of the IDU 13 from the motor space to joint angles $\vec{\theta}_s$ in the joint space by using the forward kinematics model of Equation (21). The joint space converter 622 delivers the joint angles $\vec{\theta}_s$ to the PID controller 300 and the NS controller 400. The total controller 620 may also include a distribution controller 310 as detailed below which also receives the joint angles $\vec{\theta}_s$ from the joint space controller 622. The distribution controller 310 may be a separate controller or may be integrated into the PID controller 300.

By operating the PID controller 300 directly in the joint space, the yaw, pitch, and jaw joints can have different controlled stiffnesses if desired. The output from the PID controller 300 in the joint space is joint torque $\vec{\tau}_j$ which is calculate as:

$$\vec{\tau}_j = K_p \vec{\theta}_e + K_i \int_{t_0}^{t} \vec{\theta}_e dt + K_d \frac{d}{dt} \vec{\theta}_e \qquad (65)$$

The distribution controller 310 receives the joint torques $\vec{\tau}_j$ from the PID controller 300 and distributes the joint torques $\vec{\tau}_j$ to the motor torques $\vec{\tau}_m$. However, there is not an equation that directly distributes the joint torques $\vec{\tau}_j$ to motor torques $\vec{\tau}_m$. Equation (25) relates motor torques $\vec{\tau}_m$ to joint torques $\vec{\tau}_j$ but is not invertible. Similar to the forward kinematics, a pseudoinverse of Equation (26) can be used to distribute the joint torques $\vec{\tau}_j$ to motor torques $\vec{\tau}_m$. The distribution controller 310 may account for friction when distributing the joint torques $\vec{\tau}_j$ to the motor torques $\vec{\tau}_m$. The distribution controller 310 then outputs the motor torques $\vec{\tau}_m$ to the NS controller 400. As noted above, the distribution controller 310 may be separate from or integrated into the PID controller 300.

The NS controller 400 then receives the motor torques $\vec{\tau}_m$ from the distribution controller 310 and calculates the null torques $\vec{\tau}_{null}$, adds the motor torques $\vec{\tau}_m$ and the null torques $\vec{\tau}_{null}$, and outputs the desired torque $\vec{\tau}_d$ directly to the IDU 13 in a manner similar to those detailed above.

The overall controllers 600, 610, 620 are examples of contemplated overall controllers and should not be seen as limiting. Other overall controllers are also considered to accommodate the differential drive mechanism which controllers maintain cable tension and generate clamping forces using a null space controller. For example, another overall controller may verify the desired torque $\vec{\tau}_d$ before the desired torque $\vec{\tau}_d$ is delivered to the IDU 13 to verify that the desired torque $\vec{\tau}_d$ is within an acceptable range for the IDU 13. In such a controller, if one or more of the desired torques $\vec{\tau}_d$ is outside of a range for the IDU 13, then a null space technique may be used to adjust the desired torques $\vec{\tau}_d$ before outputting the desired torques $\vec{\tau}_d$ to the IDU 13, e.g., the cable tension may be reduced and/or the clamping force may be reduced.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope of the claims appended hereto.

**Claims**

1. A controller for an end effector the end effector controlled by four cables of an open loop differential drive mechanism, the controller comprising:

primary controller configured to receive a desired pose for the end effector in yaw, pitch, and jaw degrees-of-freedom (DOF), to generate a motor torque for each motor of an instrument drive unit (IDU) to position the end effector in the desired pose, and to transmit the motor torques; **characterized in that**,
a secondary controller configured to receive the motor torques from the primary controller, to generate null torques for each of the motors of the IDU to maintain tension in the cables of the differential drive mechanism; and
an IDU configured to receive desired torques which include a sum of the motor torques and the null torques and to manipulate the end effector to the desired pose in response to receiving the desired torques.

2. A controller as claimed in claim 1 which is adapted to:

receive, in the primary controller, a desired pose of an end effector in three degrees-of-freedom (DOF);
generate in the primary controller, a motor torque for each motor of an instrument drive unit (IDU) in response to receiving the desired pose;
transmit, from the primary controller, the motor torques for each motor of the IDU;
receive, in the secondary controller, the motor torques for each motor of the IDU;
generate, in the secondary controller, a null torque for each motor of the IDU to maintain cable tension in cables of a differential drive mechanism of the IDU;
generate a desired torque for each motor of the IDU which includes a sum of the motor torques and the null torques; and
transmit the desired torques to the IDU such that the IDU moves the end effector to the desired pose.

3. The controller according to claim 2, adapted to generate the null torque for each motor of the IDU and also generate a clamping force between jaws of the end effector.

4. The controller according to claim 3 wherein being adapted to generate the clamping force includes being adapted to modify the desired pose such that a jaw angle between the jaws of the end effector is negative.

5. The controller according to claim 3 or 4, further comprising being adapted to verify a position of the jaws of the end effector is less than a clamping threshold before generating the clamping force preferably further comprising being adapted to release the clamping force when the jaws have a position greater than a releasing threshold yet further preferably wherein the releasing threshold is greater than the clamping threshold.

6. The controller according to claims 2 to 5, wherein being adapted to generate the null torque for each motor includes being adapted to receive, in the secondary controller, a sensed torque from the IDU, the sensed torque from the IDU affecting the null torque for each motor of the IDU preferably wherein being adapted to generate the null torque for each motor includes being adapted to adjust the null torque for each motor in response to a sensed torque of the respective motor.

7. The controller according to claim 6, wherein being adapted to adjust the null torque for each motor includes being adapted to apply a gain to the motor torque of each motor preferably wherein being adapted to generate the null torque for each motor includes being adapted to adjust the null torque for a puller motor for each pair of motors of the IDU in response to the sensed torques.

8. The controller according to claims 2 to 7, wherein being adapted to generate a desired torque for each motor includes being adapted to:

receive the motor torques and null torques in a tertiary controller; and
combine the motor torques and the null torques into desired torques including the sum of the motor and null torques.

9. The controller according to claim 8, wherein being adapted to transmit the desired torques to the IDU includes the tertiary controller being adapted to transmit the desired torques to the IDU preferably wherein being adapted to combine the motor torques and the null torques includes being adapted to receive sensed torques from the IDU and applying a gain to the sum of the motor and null torques to determine the desired torques such that the sensed torques approach the sum of the motor and null torques.

10. The controller according to claims 2 to 9 , wherein being adapted to generate the null torque for each motor includes being adapted to receive, in the secondary controller, a motor position for each motor of the IDU preferably wherein the motor position received by the secondary controller is in a joint space.

11. The controller according to claim 10 , further comprising being adapted to convert a motor position from a motor space to the joint space in a converter positioned between the IDU and the secondary controller.

12. The controller according to claims 2 to 11, wherein being adapted to generate the motor torque for each motor includes being adapted to calculate the motor torques in a joint space and compensating for friction preferably further comprising being adapted to distribute the motor torques in the joint space to each motor before receiving, in the secondary controller, the motor torques for each motor.

13. An instrument drive unit (IDU) adapted to control an end effector controlled by four cables of an open loop differential drive mechanism, the IDU including a controller as claimed in claim 1 and further including motors configured to receive desired torques and to manipulate the end effector to a desired pose in response to receiving the desired torques;

14. A surgical robot comprising an end effector and a controller as claimed in claim 1.

**Patentansprüche**

1. Steuerung für einen Endeffektor, wobei der Endeffektor durch vier Kabel eines Differentialantriebsmechanismus mit offenem Regelkreis gesteuert wird, die Steuerung umfassend:

eine primäre Steuerung, die konfiguriert ist, um eine gewünschte Pose für den Endeffektor in dem Gier-, Nick- und Backen-Freiheitsgrad (Gier-, Nick- und Backen-DOF) aufzunehmen, um ein Motordrehmoment für jeden Motor einer Instrumentenantriebseinheit (IDU) zu erzeugen, um den Endeffektor in der gewünschten Pose zu positionieren, und um die Motordrehmomente zu übertragen; **dadurch gekennzeichnet, dass**
eine sekundäre Steuerung, die konfiguriert ist, um die Motordrehmomente von der primären Steuerung aufzunehmen, um für jeden der Motoren der IDU Nulldrehmomente zu erzeugen, um eine Spannung in den Kabeln des Differentialantriebsmechanismus aufrechtzuerhalten; und
eine IDU, die konfiguriert ist, um gewünschte Drehmomente aufzunehmen, die eine Summe der Motordrehmomente und der Nulldrehmomente einschließen, und um den Endeffektor als Reaktion auf das Aufnehmen der gewünschten Drehmomente in die gewünschte Pose zu manipulieren.

2. Steuerung nach Anspruch 1, die angepasst ist zum:

Aufnehmen, in der primären Steuerung, einer gewünschten Pose eines Endeffektors in drei Freiheitsgraden (DOF);
Erzeugen, in der primären Steuerung, eines Motordrehmoments für jeden Motor einer Instrumentenantriebseinheit (IDU) als Reaktion auf das Aufnehmen der gewünschten Pose;
Übertragen, von der primären Steuerung, der Motordrehmomente für jeden Motor der IDU;
Aufnehmen, in der sekundären Steuerung, der Motordrehmomente für jeden Motor der IDU;
Erzeugen, in der sekundären Steuerung, eines Nulldrehmoments für jeden Motor der IDU, um die Kabelspannung in den Kabeln eines Differentialantriebsmechanismus der IDU aufrechtzuerhalten;
Erzeugen eines gewünschten Drehmoments für jeden Motor der IDU, das eine Summe der Motordrehmomente und der Nulldrehmomente einschließt; und
Übertragen der gewünschten Drehmomente an die IDU derart, dass die IDU den Endeffektor in die gewünschte Pose bewegt.

3. Steuerung nach Anspruch 2, die angepasst, um das Nulldrehmoment für jeden Motor der IDU zu erzeugen und auch eine Klemmkraft zwischen den Backen des Endeffektors zu erzeugen.

4. Steuerung nach Anspruch 3, wobei das Angepasstwerden, um die Klemmkraft zu erzeugen, das Angepasstwerden einschließt, um die gewünschte Pose derart zu modifizieren, dass ein Backenwinkel zwischen den Backen des Endeffektors negativ ist.

5. Steuerung nach Anspruch 3 oder 4, ferner umfassend das Angepasstwerden, um zu überprüfen, dass eine Position der Backen des Endeffektors unter einem Klemmschwellenwert liegt, bevor die Klemmkraft erzeugt wird, vorzugsweise ferner umfassend das Angepasstwerden, um die Klemmkraft freizugeben, wenn die Backen eine Position aufweisen, die über einem Freigabeschwellenwert liegt, weiter bevorzugt, wobei der Freigabeschwellenwert größer als der Klemmschwellenwert ist.

6. Steuerung nach den Ansprüchen 2 bis 5, wobei das Angepasstwerden, um das Nulldrehmoment für jeden Motor zu erzeugen, das Angepasstwerden einschließt, um ein erfasstes Drehmoment von der IDU in der sekundären Steuerung aufzunehmen, wobei das erfasste Drehmoment von der IDU das Nulldrehmoment für jeden Motor der IDU beeinflusst, vorzugsweise wobei das Angepasstwerden, um das Nulldrehmoment für jeden Motor zu erzeugen, das Angepasstwerden einschließt, um das Nulldrehmoment für jeden Motor als Reaktion auf ein erfasstes Drehmoment des jeweiligen Motors einzustellen.

7. Steuerung nach Anspruch 6, wobei das Angepasstwerden, um das Nulldrehmoment für jeden Motor einzustellen, das Angepasstwerden einschließt, um eine Verstärkung auf das Motordrehmoment jedes Motors auszuüben, vorzugsweise wobei das Angepasstwerden, um das Nulldrehmoment für jeden Motor zu erzeugen, das Angepasstwerden einschließt, um das Nulldrehmoment für einen Zugmotor für jedes Paar von Motoren der IDU als Reaktion auf die erfassten Drehmomente einzustellen.

8. Steuerung nach den Ansprüchen 2 bis 7, wobei das Angepasstwerden, um ein gewünschtes Drehmoment für jeden Motor das Angepasstwerden einschließt zum:

   Aufnehmen der Motordrehmomente und der Nulldrehmomente in einer tertiären Steuerung; und
   Kombinieren der Motordrehmomente und der Nulldrehmomente zu gewünschten Drehmomenten, einschließlich der Summe der Motor- und der Nulldrehmomente.

9. Steuerung nach Anspruch 8, wobei das Angepasstwerden, um die gewünschten Drehmomente an die IDU zu übertragen, einschließt, dass die tertiäre Steuerung angepasst wird, um die gewünschten Drehmomente an die IDU zu übertragen, vorzugsweise wobei das Angepasstwerden, um die Motordrehmomente und die Nulldrehmomente zu kombinieren, das Angepasstwerden, um die erfassten Drehmomente von der IDU aufzunehmen, und das Anwenden einer Verstärkung auf die Summe der Motor- und der Nulldrehmomente einschließt, um die gewünschten Drehmomente derart zu bestimmen, dass die erfassten Drehmomente sich der Summe der Motor- und der Nulldrehmomente annähern.

10. Steuerung nach den Ansprüchen 2 bis 9, wobei das Angepasstwerden, um das Nulldrehmoment für jeden Motor das Angepasstwerden, um eine Motorposition für jeden Motor der IDU in der sekundären Steuerung aufzunehmen, einschließt, vorzugsweise wobei die Motorposition, die durch die sekundäre Steuerung aufgenommen wird, sich in einem Gelenkraum befindet.

11. Steuerung nach Anspruch 10, ferner umfassend das Angepasstwerden, um eine Motorposition von einem Motorraum in den Gelenkraum in einem Konverter umzuwandeln, der zwischen der IDU und der sekundären Steuerung positioniert ist.

12. Steuerung nach den Ansprüchen 2 bis 11, wobei das Angepasstwerden, um das Motordrehmoment für jeden Motor das Angepasstwerden, um die Motordrehmomente in einem Gelenkraum zu berechnen, und ein Kompensieren der Reibung einschließt, vorzugsweise ferner umfassend das Angepasstwerden, um die Motordrehmomente in dem Gelenkraum auf jeden Motor zu verteilen, bevor in der sekundären Steuerung die Motordrehmomente für jeden Motor aufgenommen werden.

13. Instrumentenantriebseinheit (IDU), die angepasst ist, um einen Endeffektor zu steuern, der durch vier Kabel eines Differentialantriebsmechanismus mit offenem Regelkreis gesteuert wird, wobei die IDU eine Steuerung nach Anspruch 1 umfasst und ferner Motoren einschließt, die konfiguriert sind, um gewünschte Drehmomente aufzunehmen und um den Endeffektor als Reaktion auf das Aufnehmen der gewünschten Drehmomente in eine gewünschte Pose zu manipulieren;

14. Chirurgischer Roboter, umfassend einen Endeffektor und eine Steuerung nach Anspruch 1.

**Revendications**

1. Dispositif de commande pour un effecteur terminal, l'effecteur terminal commandé par quatre câbles d'un mécanisme d'entraînement différentiel en boucle ouverte, le dispositif de commande comprenant :

   un dispositif de commande primaire configuré pour recevoir une position souhaitée pour l'effecteur terminal dans les degrés de liberté (DOF) lacet, tangage et mâchoire, pour générer un couple moteur pour chaque moteur d'une unité d'entraînement d'instrument (IDU) afin de positionner l'effecteur terminal dans la position souhaitée, et pour transmettre les couples moteurs ;
   **caractérisé en ce que,**
   un dispositif de commande secondaire configuré pour recevoir les couples moteurs du dispositif de commande primaire, pour générer des couples nuls pour chacun des moteurs de l'IDU afin de maintenir la tension dans les câbles du mécanisme d'entraînement différentiel ; et
   une IDU configurée pour recevoir les couples souhaités, qui comportent une somme des couples moteurs et des couples nuls, et pour manipuler l'effecteur terminal dans la position souhaitée en réponse à la réception des couples souhaités.

2. Dispositif de commande selon la revendication 1, qui est destiné à :

   recevoir, dans le dispositif de commande primaire, une position souhaitée d'un effecteur terminal dans trois degrés de liberté (DOF) ;
   générer, dans le dispositif de commande primaire, un couple moteur pour chaque moteur d'une unité d'entraînement d'instrument (IDU) en réponse à la réception de la position souhaitée ;
   transmettre, à partir du dispositif de commande primaire, les couples moteurs pour chaque moteur de l'IDU ;
   recevoir, dans le dispositif de commande secondaire, les couples moteurs pour chaque moteur de l'IDU ;
   générer, dans le dispositif de commande secondaire, un couple nul pour chaque moteur de l'IDU afin de maintenir la tension de câble dans des câbles d'un mécanisme d'entraînement différentiel de l'IDU ;
   générer un couple souhaité pour chaque moteur de l'IDU, qui comporte une somme des couples moteurs et des couples nuls ; et
   transmettre les couples souhaités à l'IDU de telle sorte que l'IDU déplace l'effecteur terminal dans la position souhaitée.

3. Dispositif de commande selon la revendication 2, destiné à générer le couple nul pour chaque moteur de l'IDU et générer également une force de serrage entre des mâchoires de l'effecteur terminal.

4. Dispositif de commande selon la revendication 3, dans lequel le fait d'être destiné à générer la force de serrage comporte le fait d'être destiné à modifier la position souhaitée de telle sorte qu'un angle de mâchoire entre les mâchoires de l'effecteur terminal soit négatif.

5. Dispositif de commande selon la revendication 3 ou 4, comprenant en outre le fait d'être destiné à vérifier qu'une position des mâchoires de l'effecteur terminal est inférieure à un seuil de serrage avant de générer la force de serrage, de préférence comprenant en outre le fait d'être destiné à relâcher la force de serrage lorsque les mâchoires ont une position supérieure à un seuil de relâchement, de préférence encore dans lequel le seuil de relâchement est supérieur au seuil de serrage.

6. Dispositif de commande selon les revendications 2 à 5, dans lequel le fait d'être destiné à générer le couple nul pour chaque moteur comporte le fait d'être destiné à recevoir, dans le dispositif de commande secondaire, un couple détecté de l'IDU, le couple détecté de l'IDU affectant le couple nul pour chaque moteur de l'IDU, de préférence dans lequel le fait d'être destiné à générer le couple nul pour chaque moteur comporte le fait d'être destiné à ajuster le couple nul pour chaque moteur en réponse à un couple détecté du moteur respectif.

7. Dispositif de commande selon la revendication 6, dans lequel le fait d'être destiné à ajuster le couple nul pour chaque moteur comporte le fait d'être destiné à appliquer un gain au couple moteur de chaque moteur, de préférence dans lequel le fait d'être destiné à générer le couple nul pour chaque moteur comporte le fait d'être destiné à ajuster le couple nul pour un moteur de traction pour chaque paire de moteurs de l'IDU en réponse aux couples détectés.

8. Dispositif de commande selon les revendications 2 à 7, dans lequel le fait d'être destiné à générer un couple souhaité pour chaque moteur comporte le fait d'être destiné à :

recevoir les couples moteurs et les couples nuls dans un dispositif de commande tertiaire ; et

combiner les couples moteurs et les couples nuls en couples souhaités, y compris la somme des couples moteurs et des couples nuls.

9. Dispositif selon la revendication 8, dans lequel le fait d'être destiné à transmettre les couples souhaités à l'IDU comporte le fait que le dispositif de commande tertiaire est destiné à transmettre les couples souhaités à l'IDU, de préférence dans lequel le fait d'être destiné à combiner les couples moteurs et les couples nuls comporte le fait d'être destiné à recevoir les couples détectés de l'IDU et d'appliquer un gain à la somme des couples moteurs et des couples nuls pour déterminer les couples souhaités de telle sorte que les couples détectés se rapprochent de la somme des couples moteurs et des couples nuls.

10. Dispositif de commande selon les revendications 2 à 9, dans lequel le fait d'être destiné à générer le couple nul pour chaque moteur comporte le fait d'être destiné à recevoir, dans le dispositif de commande secondaire, une position de moteur pour chaque moteur de l'IDU, de préférence dans lequel la position de moteur reçue par le dispositif de commande secondaire est dans un espace d'articulation.

11. Dispositif de commande selon la revendication 10, comprenant en outre le fait d'être destiné à convertir une position de moteur d'un espace moteur à l'espace d'articulation dans un convertisseur positionné entre l'IDU et le dispositif de commande secondaire.

12. Dispositif de commande selon les revendications 2 à 11, dans lequel le fait d'être destiné à générer le couple moteur pour chaque moteur comporte le fait d'être destiné à calculer les couples moteurs dans un espace d'articulation et à compenser le frottement, de préférence comprenant en outre le fait d'être destiné à distribuer les couples moteurs dans l'espace d'articulation à chaque moteur avant de recevoir, dans le dispositif de commande secondaire, les couples moteurs pour chaque moteur.

13. Unité d'entraînement d'instrument (IDU) destinée à commander un effecteur terminal commandé par quatre câbles d'un mécanisme d'entraînement différentiel en boucle ouverte, l'IDU comportant un dispositif de commande selon la revendication 1 et comportant en outre des moteurs conçus pour recevoir des couples souhaités et pour manipuler l'effecteur terminal dans une position souhaitée en réponse à la réception des couples souhaités ;

14. Robot chirurgical comprenant un effecteur terminal et un dispositif de commande selon la revendication 1.

*FIG. 1*

FIG. 2

FIG. 3

FIG. 4

FIG. 5

FIG. 6    FIG. 7    FIG. 8    FIG. 9    FIG. 10

EP 3 890 640 B1

FIG. 11

FIG. 12

FIG. 13

FIG. 14

## REFERENCES CITED IN THE DESCRIPTION

**Patent documents cited in the description**

- US 62776285
- US 8828023 B
- US 20180153634 A